(19) Europäisches Patentamt
European Patent Office
Office européen des brevets

(11) Veröffentlichungsnummer : **0 391 187 B1**

# (12) EUROPÄISCHE PATENTSCHRIFT

(45) Veröffentlichungstag der Patentschrift : **11.11.92 Patentblatt 92/46**

(21) Anmeldenummer : **90105654.9**

(22) Anmeldetag : **24.03.90**

(51) Int. Cl.5 : **C07D 249/12,** A01N 43/653, C07C 265/10, C07C 211/37, C07C 61/15, C07C 69/74, C07C 233/58

## (54) Substituierte 4-Amino-5-alkylthio-1,2,4-triazol-3-one.

Die Akte enthält technische Angaben, die nach dem Eingang der Anmeldung eingereicht wurden und die nicht in dieser Patentschrift enthalten sind.

(30) Priorität : **07.04.89 DE 3911219**
**10.10.89 DE 3933750**

(43) Veröffentlichungstag der Anmeldung : **10.10.90 Patentblatt 90/41**

(45) Bekanntmachung des Hinweises auf die Patenterteilung : **11.11.92 Patentblatt 92/46**

(84) Benannte Vertragsstaaten : **BE CH DE FR GB IT LI NL**

(56) Entgegenhaltungen :
**EP-A- 0 294 666**
**Patent Abstracts of Japan, Band 2, Nr. 24, 16. Februar 1978, Seite 4198; & JP-A-52 125 168 (Nippon Soda K.K.) 20.10.1977**
**Chemical Abstracts, Band 90, Nr. 19, 7. Mai 1979, Seite 617, Columbus, Ohio, USA; & JP-A-78 135 981 (Nippon Soda Co.Ltd.)**

(73) Patentinhaber : **BAYER AG**
**W-5090 Leverkusen 1 Bayerwerk (DE)**

(72) Erfinder : **Müller, Klaus-Helmut, Dr.**
**Bockhackstrasse 55**
**W-4000 Düsseldorf 13 (DE)**
Erfinder : **Kluth, Joachim, Dr.**
**Tannenweg 9**
**W-4018 Langenfeld (DE)**
Erfinder : **König, Klaus, Dr.**
**Zum Hahnberg 40**
**W-5068 Odenthal (DE)**
Erfinder : **Gassen, Karl-Rudolf, Dr.**
**Auenweg 6a**
**W-5068 Odenthal (DE)**
Erfinder : **Findeisen, Kurt, Dr.**
**Dünfelder Strasse 28**
**W-5090 Leverkusen 1 (DE)**
Erfinder : **Lindig, Markus, Dr.**
**Hildegardstrasse 9**
**W-4018 Langenfeld (DE)**
Erfinder : **Lürssen, Klaus, Dr.**
**August-Kierspel-Strasse 145**
**W-5060 Leverkusen 1 (DE)**
Erfinder : **Santel, Hans-Joachim, Dr.**
**Grünstrasse 9a**
**W-5090 Leverkusen 1 (DE)**
Erfinder : **Schmidt, Robert R., Dr.**
**Im Waldwinkel 110**
**W-5060 Bergisch Gladbach 2 (DE)**

EP 0 391 187 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue substituierte 4-Amino-5-alkylthio-1,2,4-triazol-3-one, mehrere Verfahren und neue Zwischenprodukte zu ihrer Herstellung sowie ihre Verwendung als Herbizide.

Es ist bekannt, daß bestimmte 4-Amino-5-alkylthio-1,2,4-triazol-3-one, wie z.B. 4-Amino-5-methylthio-2-cyclohexylaminocarbonyl-2,4-dihydro-3H-1,2,4-triazol-3-on, 4-Amino-5-methylthio-2-isopropylaminocarbonyl-2,4-dihydro-3H-1,2,4-triazol-3-on 4-Amino-5-methylthio-2-butylamino-carbonyl-2,4-dihydro-3H-1,2,4-triazol-3-on, herbizid wirksam sind (vgl. JP 52-125168). Die Wirkung dieser bekannten Verbindungen ist jedoch nicht in allen Belangen zufriedenstellend. Weitere Triazolderivate mit herbizider Wirkung werden in der EP-A-0 294 666 beschrieben.

Es wurden nun die neuen substituierten 4-Amino-5-alkylthio-1,2,4-triazol-3-one der allgemeinen Formel (I)

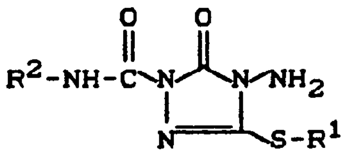

(I)

in welcher

$R^1$ für jeweils geradkettiges oder verzweigtes Alkyl, Alkenyl oder Alkinyl mit jeweils bis zu 4 Kohlenstoffatomen steht und

$R^2$ für sec.-Butyl, tert.-Butyl, jeweils geradkettiges oder verzweigtes $C_5$-$C_{10}$-Alkyl, $C_5$-$C_{10}$-Alkenyl oder $C_5$-$C_{10}$-Alkinyl, für jeweils durch Halogen, Cyano, $C_3$-$C_6$-Cycloalkyl, Aryloxy mit 6 bis 10 Kohlenstoffatomen (insbesondere Phenyl oder Naphthyl) oder $C_1$-$C_6$-Alkoxy substituiertes, geradkettiges oder verzweigtes Alkyl, Alkenyl oder Alkinyl mit jeweils bis zu 10 Kohlenstoffatomen, für jeweils durch Piperidyl oder Morpholinyl substituiertes $C_1$-$C_6$-Alkyl, für gegebenenfalls durch Halogen, Aryl mit 6 bis 10 Kohlenstoffatomen (insbesondere Phenyl oder Naphthyl), $C_1$-$C_6$-Alkyl oder $C_1$-$C_4$-Halogenalkyl substituiertes Cyclopropyl, Cyclobutyl, Cyclopentyl oder Cycloheptyl, für durch Halogen, $C_1$-$C_6$-Alkyl, $C_2$-$C_4$-Alkenyl oder $C_1$-$C_4$-Halogenalkyl substituiertes Cyclohexyl, für jeweils gegebenenfalls in der aromatischen Komponente durch Halogen, $C_1$-$C_4$-Alkyl, $C_1$-$C_2$-Halogenalkyl, $C_1$-$C_4$-Alkoxy, $C_1$-$C_2$-Halogen alkoxy, $C_1$-$C_4$-Alkylthio, $C_1$-$C_4$-Alkylsulfinyl, $C_1$-$C_4$-Alkylsulfonyl, Phenoxy und/oder Phenyl substituiertes Phenyl-$C_1$-$C_2$-alkyl oder Naphthyl-$C_1$-$C_2$-alkyl steht,

gefunden.

Die einzelnen Kohlenstoffketten in z.B. Alkyl, Halogenalkyl oder Alkenyl sind jeweils geradkettig oder verzweigt.

Weiter wurde gefunden, daß man die neuen substituierten 4-Amino-5-alkylthio-1,2,4-triazol-3-one der allgemeinen Formel (I)

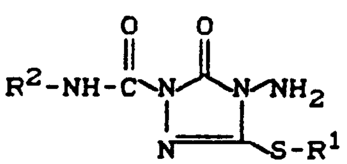

(I)

in welcher

$R^1$ für jeweils geradkettiges oder verzweigtes Alkyl, Alkenyl oder Alkinyl mit jeweils bis zu 4 Kohlenstoffatomen steht und

$R^2$ für sec.-Butyl, tert.-Butyl, jeweils geradkettiges oder verzweigtes $C_5$-$C_{10}$-Alkyl, $C_5$-$C_{10}$-Alkenyl oder $C_5$-$C_{10}$-Alkinyl, für jeweils durch Halogen, Cyano, $C_3$-$C_6$-Cycloalkyl, Aryloxy mit 6 bis 10 Kohlenstoffatomen (insbesondere Phenyl oder Naphthyl) oder $C_1$-$C_6$-Alkoxy substituiertes, geradkettiges oder verzweigtes Alkyl, Alkenyl oder Alkinyl mit jeweils bis zu 10 Kohlenstoffatomen, für jeweils durch Piperidyl oder Morpholinyl substituiertes $C_1$-$C_6$-Alkyl, für gegebenenfalls durch Halogen, Aryl mit 6 bis 10 Kohlenstoffatomen (insbesondere

Phenyl oder Naphthyl), $C_1$-$C_6$-Alkyl, oder $C_1$-$C_4$-Halogenalkyl substituiertes Cyclopropyl, Cyclobutyl, Cyclopentyl oder Cycloheptyl, für durch Halogen, $C_1$-$C_6$-Alkyl, $C_2$-$C_4$-Alkenyl oder $C_1$-$C_4$-Halogenalkyl substituiertes Cyclohexyl, für jeweils gegebenenfalls in der aromatischen Komponente durch Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_4$-Alkylsulfinyl, $C_1$-$C_4$-Alkylsulfonyl, Phenoxy, und/oder Phenyl substituiertes Phenyl-$C_1$-$C_2$-alkyl oder Naphthyl-$C_1$-$C_2$-alkyl steht,

erhält, wenn man

(a) Hydrazone der allgemeinen Formel (II)

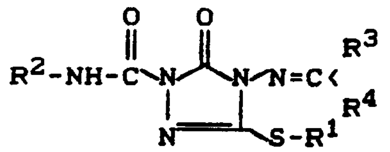

(II)

in welcher

$R^1$ und $R^2$ die oben angegebenen Bedeutungen haben und

$R^3$ und $R^4$ unabhängig voneinander jeweils für Wasserstoff, $C_1$-$C_6$-Alkyl, Phenyl-$C_1$-$C_4$-alkyl oder Phenyl stehen,

mit einer Säure, gegebenenfalls in Gegenwart eines Verdünnungsmittels umsetzt, oder wenn man

(b) Triazolone der allgemeinen Formel (III)

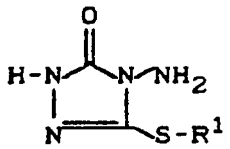

(III)

in welcher

$R^1$ die oben angegebene Bedeutung hat,

mit Isocyanaten der allgemeinen Formel (IV)

$$R^2 - N = C = O \quad (IV)$$

in welcher

$R^2$ die oben angegebene Bedeutung hat,

gegebenenfalls in Gegenwart eines Verdünnungsmittels und gegebenenfalls in Gegenwart eines Reaktionshilfsmittels umsetzt, oder wenn man

(c) Oxycarbonyltriazolone der allgemeinen Formel (V)

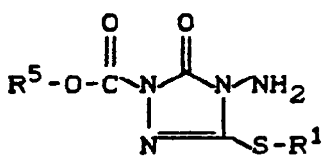

(V)

in welcher

$R^1$ die oben angegebene Bedeutung hat und

$R^5$ für $C_1$-$C_6$-Alkyl, Phenyl oder Phenyl-$C_1$-$C_4$-alkyl steht,

mit Aminen der allgemeinen Formel (VI)

$$R^2 - NH_2 \quad (VI)$$

in welcher

$R^2$ die oben angegebene Bedeutung hat,

gegebenenfalls in Gegenwart eines Verdünnungsmittels und gegebenenfalls in Gegenwart eines Reaktionshilfsmittels umsetzt.

Schließlich wurde gefunden, daß die neuen substituierten 4-Amino-5-alkylthio-1,2,4-triazol-3-one der allgemeinen Formel (I) interessante herbizide Eigenschaften aufweisen.

Überraschenderweise zeigen die erfindungsgemäßen substituierten 4-Amino-5-alkylthio-1,2,4-triazol-3-one der allgemeinen Formel (I) erheblich stärkere Herbizidwirkung gegen Problemunkräuter als die bekannten herbizid wirksamen Verbindungen 4-Amino-5-methylthio-2-cyclohexylaminocarbonyl-2,4-dihydro-3H-1,2,4-triazol-3-on, 4-Amino-5-methylthio-2-isopropylaminocarbonyl-2,4-dihydro-3H-1,2,4-triazol-3-on und 4-Amino-5-methylthio-2-butylaminocarbonyl-2,4-dihydro-3H-1,2,4-triazol-3-on, welche strukturell naheliegende Stoffe sind.

Die Erfindung betrifft vorzugsweise Verbindungen der Formel (I), in welcher

$R^1$ für Methyl, Ethyl, Propyl, Isopropyl, Butyl, Isobutyl, sec.-Butyl, tert.-Butyl, Allyl, Crotonyl oder Propargyl steht und

$R^2$ für sec.-Butyl, tert.-Butyl, jeweils geradkettiges oder verzweigtes Pentyl, Hexyl, Heptyl, Octyl, Pentenyl, Hexenyl, Heptenyl, Octenyl, Pentinyl, Hexinyl, Heptinyl oder Octinyl, für jeweils durch Fluor, Chlor, Brom, Cyano, Cyclopropyl, Cyclobutyl, Cyclopentyl, Cyclohexyl, Phen-oxy, Methoxy, Ethoxy, Propoxy, Isopropoxy, Butoxy, Isobutoxy, sec.-Butoxy oder tert.-Butoxy substituiertes Methyl, Ethyl, Propyl, Isopropyl, Butyl, Isobutyl, sec.-Butyl, tert.-Butyl, Pentyl, Isopentyl, sec.-Pentyl, tert.-Pentyl, Allyl, Crotonyl oder Propargyl steht, oder $R^2$ weiterhin für jeweils durch Piperidinyl oder Morpholinyl substituiertes Methyl, Ethyl, n- oder iso-Propyl, für gegebenenfalls jeweils durch Fluor, Chlor, Brom, Methyl, Trifluormethyl, Ethyl, Propyl, Isopropyl, Butyl, Isobutyl, sec.-Butyl oder tert.-Isobutyl, sec.-Butyl oder tert.-Butyl substituiertes Cyclopropyl, Cyclobutyl oder Cyclopentyl, für durch Fluor, Chlor, Methyl, Trifluormethyl, Ethyl, Propyl, Isopropyl, Butyl, Isobutyl, sec.-Butyl, tert.-Butyl oder Propargyl substituiertes Cyclohexyl steht, $R^2$ ferner für jeweils gegebenenfalls in der aromatischen Komponente durch Fluor, Chlor, Brom, Methyl, Ethyl, Propyl, Isopropyl, Trifluormethyl, Methoxy, Ethoxy, Difluormethoxy, Trifluormethoxy, Methylthio, Methylsulfinyl, Methylsulfonyl, Phenoxy und/oder Phenyl substituiertes Benzyl oder Phenylethyl steht.

Die Erfindung betrifft insbesondere Verbindungen der Formel (I), in welcher

$R^1$ für Methyl, Ethyl, Propyl oder Allyl steht, und

$R^2$ für sec.-Butyl, tert.-Butyl, jeweils geradkettiges oder verzweigtes Pentyl, Hexyl, Heptyl, Pentenyl, Hexenyl, Heptenyl, Pentinyl, Hexinyl oder Heptinyl, für jeweils durch Fluor, Chlor, Methoxy, Phenoxy oder Cyano substituiertes Methyl, Ethyl, Propyl, Isopropyl, Butyl, Isobutyl, sec.-Butyl oder tert.-Butyl, für jeweils gege-benenfalls durch Fluor, Chlor, Methyl, Trifluormethyl, Ethyl, Propyl, Isopropyl, Butyl, Isobutyl, sec.-Butyl oder tert.-Butyl substituiertes Cyclopropyl, Cyclobutyl oder Cyclopentyl, für durch Chlor, Methyl, Trifluormethyl, Ethyl, Propyl, Isopropyl, Butyl, Isobutyl, sec.-Butyl oder tert.-Butyl substituiertes Cyclohexyl, für gegebenenfalls in der aromatischen Komponente durch Fluor, Chlor, Brom, Methyl, Ethyl, Methoxy, Ethoxy und/oder Phenyl substituiertes (R/S)-, (R) oder (S)-1-Phenylethyl steht.

Verwendet man beispielsweise 4-Isopropylidenimino-5-ethylthio-2-tert.-butylaminocarbonyl-2,4-dihydro-3H-1,2,4-triazol-3-on als Ausgangsverbindung, so läßt sich der Reaktionsablauf des erfindungsgemäßen Verfahren (a) durch das folgende Formelschema wiedergeben:

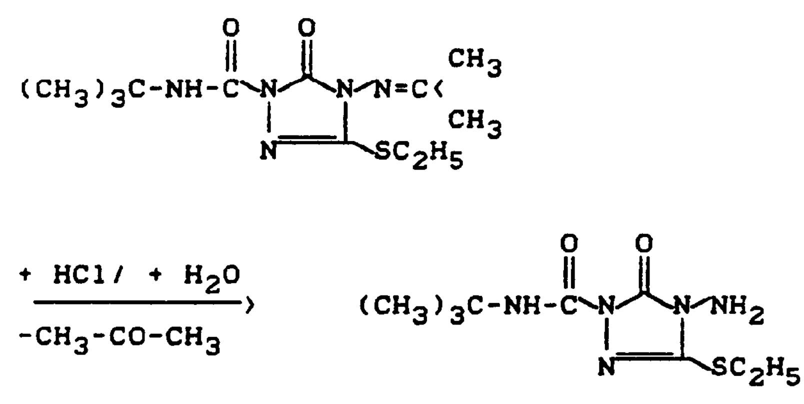


Verwendet man beispielsweise 4-Amino-5-ethylthio-2,4-dihydro-3H-1,2,4-triazol-3-on und tert.-Butylisocyanat als Ausgangsstoffe, so läßt sich der Reaktionsablauf des erfindungsgemäßen Verfahren (b) durch das folgende Formelschema wiedergeben:

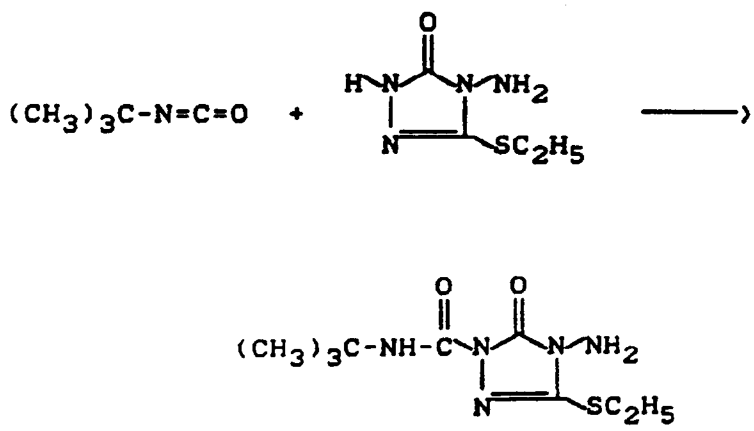

Verwendet man beispielsweise 4-Amino-5-ethylthio-2-methoxycarbonyl-2,4-dihydro-3H-1,2,4-triazol-3-on und 2-Cyano-ethylamin als Ausgangsstoffe, so läßt sich der Reaktionsablauf des erfindungsgemäßen Verfahren (c) durch das folgende Formelschema wiedergeben:

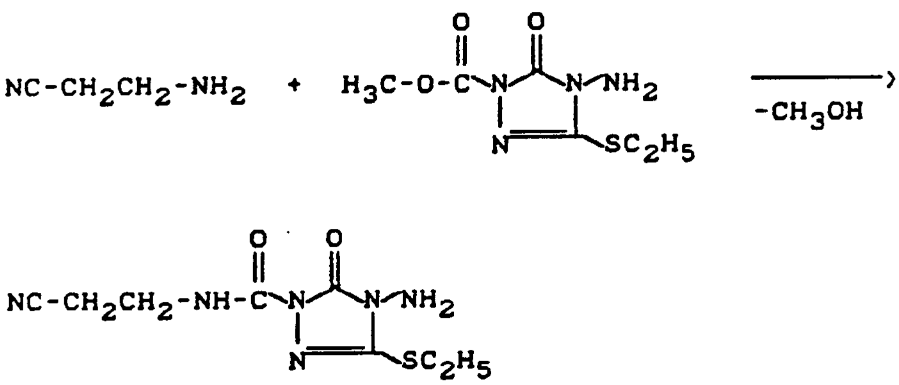


Die beim erfindungsgemäßen Verfahren (a) zur Herstellung von Verbindungen der Formel (I) als Ausgangsstoffe zu verwendenden Hydrazone sind durch die Formel (II) allgemein definiert.

In Formel (II) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden;

$R^3$ und $R^4$ stehen vorzugsweise jeweils unabhängig voneinander für Wasserstoff, für geradkettiges oder verzweigtes Alkyl mit 1 bis 4 Kohlenstoffatomen oder für Phenyl oder Benzyl, insbesondere für Wasserstoff, Methyl, Ethyl, Phenyl oder Benzyl.

Die Hydrazone der Formel (II) sind noch nicht bekannt. Sie sind ebenfalls Gegenstand der vorliegenden Erfindung. Man erhält sie jedoch in Analogie zu bekannten Verfahren (vergl. z.B. Acta Pol. Pharm. 38, 153-162 [1981] bzw. C.A. 95: 203841j), beispielsweise wenn man Triazolone der Formel (III),

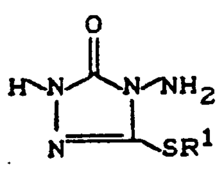 (III)

in welcher

$R^1$ die oben angegebene Bedeutung hat,

mit Aldehyden oder Ketonen der Formel (VII),

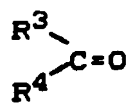 (VII)

in welcher
$R^3$ und $R^4$ die oben angegebene Bedeutung haben,
gegebenenfalls in Gegenwart eines Verdünnungsmittels wie beispielsweise Dichlormethan oder Toluol und gegebenenfalls in Gegenwart eines Katalysators wie beispielsweise p-Toluolsulfonsäure bei Temperaturen zwischen 40°C und 120°C umsetzt und die so erhältlichen Triazolon-Hydrazone der Formel (VIII),

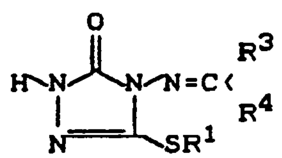 (VIII)

in welcher
$R^1$, $R^3$ und $R^4$ die oben angegebene Bedeutung haben,
entweder in einer anschließenden 2.Stufe mit Isocyanaten der Formel (IV),

$R^2$ - N = C = O (IV)

in welcher
$R^2$ die oben angegebene Bedeutung hat,
gegebenenfalls in Gegenwart eines Verdünnungsmittels wie beispielsweise Dichlormethan oder Dioxan und gegebenenfalls in Gegenwart eines Reaktionshilfsmittels wie beispielsweise Triethylamin bei Temperaturen zwischen 20°C und 150°C umsetzt;
oder alternativ in einer anschließenden 2. Stufe mit Chlorameisensäureestern der Formel (IX),

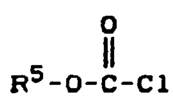 (IX)

in welcher
$R^5$ die oben angegebene Bedeutungen hat,
gegebenenfalls in Gegenwart eines Verdünnungsmittels wie beispielsweise Tetrahydrofuran und gegebenenfalls in Gegenwart eines Reaktionshilfsmittels wie beispielsweise Natriumhydrid oder Kalium-tert.-butylat bei Temperaturen zwischen - 20 °C und + 40 °C umsetzt und die so erhältlichen Triazolone der Formel (X),

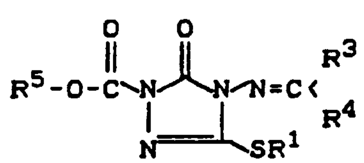 (X)

in welcher
$R^1$, $R^3$, $R^4$ und $R^5$ die oben angegebene Bedeutung haben,
in einer anschließenden 3. Stufe mit Aminen der Formel (VI),

$R^2 - NH_2$ (VI)

in welcher
$R^2$ die oben angegebene Bedeutung hat,
gegebenenfalls in Gegenwart eines Verdünnungsmittels wie beispielsweise Tetrahydrofuran oder Dioxan sowie gegebenenfalls in Gegenwart einer Base wie beispielsweise Natriumhydroxid oder Kaliumhydroxid bei Temperaturen zwischen 20 °C und 100 °C umsetzt.

Dabei ist es auch möglich und gegebenenfalls von Vorteil die Umsetzung der Triazolon-Hydrazone der Formel (VIII) mit Chlorameisensäureestern der Formel (IX) und die anschließende Umsetzung der so erhältlichen Triazolone (X) mit Aminen der Formel (VI) in einem sogenannten Eintopfverfahren durchzuführen .

Die Triazolone der Formel (III) sind bekannt oder erhältlich in Analogie zu bekannten Verfahren (vergl. z.B. JP 52-125168).

Die Aldehyde oder Ketone der Formel (VII), die Isocyanate der Formel (IV), die Chlorameisensäureester der Formel (IX) und die Amine der Formel (VI) sind weitgehend bekannte organische Synthesechemikalien.

Die Triazolon-Hydrazone der Formel (VIII) und die Triazolone der Formel (X) sind noch nicht aus der Literatur bekannt und Gegenstand der vorliegenden Patentanmeldung.

In den Formeln (VIII) und (X) haben $R^1$, $R^3$ und $R^4$ vorzugsweise bzw. insbesondere diejenige Bedeutung, die oben im Zusammenhang mit der Beschreibung der erfindungsgemäßen Verbindungen der Formeln (I) und (II) vorzugsweise bzw. als insbesondere bevorzugt für $R^1$, $R^3$ und $R^4$ angegeben wurde. $R^5$ steht vorzugsweise für Methyl, Ethyl, Propyl, Isopropyl, Butyl, Isobutyl, sec-Butyl, tert-Butyl, Phenyl oder Benzyl.

Die beim erfindungsgemäßen Verfahren (b) zur Herstellung von Verbindungen der Formel (I) als Ausgangsstoffe zu verwendenden Triazolone sind durch die Formel (III) allgemein definiert.

In Formel (III) hat $R^1$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^1$ angegeben wurde.

Die Triazolone der Formel (III) sind bekannt oder erhältlich in Analogie zu bekannten Verfahren (vgl. z.B. JP 52-125168).

Die zur Durchführung des erfindungsgemäßen Verfahrens (b) weiterhin als Ausgangsstoffe benötigten Isocyanate sind durch die Formel (IV) allgemein definiert. In dieser Formel (IV) steht $R^2$ vorzugsweise für diejenigen Reste, die bereits in Zusammenhang mit der Beschreibung der erfindungsgemäßen Stoffe der Formel (I) als bevorzugt für $R^2$ genannt wurden.

Die Isocyanate der Formel (IV) sind weitgehend bekannte organische Synthesechemikalien.

Die beim erfindungsgemäßen Verfahren (c) zur Herstellung von Verbindungen der Formel (I) als Ausgangsstoffe zu verwendenden Oxycarbonyltriazolone sind durch die Formel (V) allgemein definiert.

In Formel (V) haben $R^1$ und $R^5$ vorzugsweise bzw. insbesondere diejenigen Bedeutungen, die bereits oben im Zusammenhang mit der Beschreibung der erfindungsgemäßen Verbindungen der Formeln (I) und (VIII) vorzugsweise bzw. als insbesondere bevorzugt für $R^1$ und bevorzugt für $R^5$ angegeben wurden.

Die Oxycarbonyltriazolone der Formel (V) sind noch nicht aus der Literatur bekannt und Gegenstand der vorliegenden Patentanmeldung.

Man erhält die neuen Verbindungen der Formel (V), wenn man Triazolone der Formel (III)

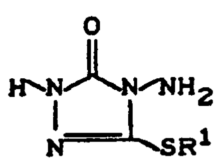

(III)

in welcher

$R^1$ die oben angegebene Bedeutung hat,

mit Chlorameisensäureestern der Formel (IX)

$$R^5\text{-O-}\overset{O}{\overset{\|}{C}}\text{-Cl} \qquad (IX)$$

in welcher

$R^5$ die oben angegebene Bedeutung hat,

gegebenenfalls in Gegenwart eines Verdünnungsmittels, wie z.B. Tetrahydrofuran, und gegebenenfalls in Gegenwart eines Reaktionshilfsmittels, wie z.B. Kalium-tertbutylat oder Natriumhydrid, bei Temperaturen

zwischen -20° C und +100°C umsetzt.

Neu sind ferner auch die Verbindungen 2-Chlor-1-methyl-2,3,3-trifluor-cyclobut-1-yl-isocyanat der Formel (IVa)

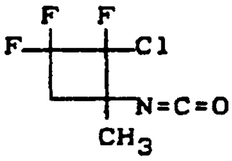

(IVa)

und 2-Chlor-1-methyl-2,3,3-trifluor-cyclobut-1-yl-amin der Formel (VIa)

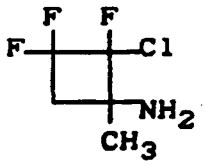

(VIa)

welche als Zwischenprodukte zur Herstellung von entsprechenden Verbindungen der Formel (I) eingesetzt werden können.

Man erhält die neue Verbindung der Formel (IVa), wenn man 2-Chlor-1-methyl-2,3,3-trifluor-cyclobutan-1-carbonsäurechlorid der Formel (X)

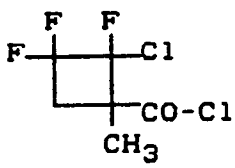

(X)

mit Trimethylsilylazid, vorzugsweise in Gegenwart eines inerten Verdünnungsmittels, wie z.B. Toluol, bei Temperaturen zwischen 60° C und 80° C umsetzt und das Produkt durch Vakuumdestillation isoliert.

Die Verbindung der Formel (IVa) kann auch aus der Verbindung der Formel (VIa) nach üblichen Methoden, beispielsweise durch Phosgenierung, hergestellt werden.

Man erhält die neue Verbindung der Formel (X) wenn man 2-Chlor-1-Methyl-2,3,3-trifluor-cyclobutan-1-carbonsäure der Formel (XI)

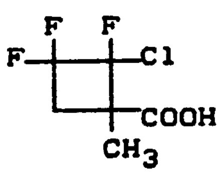

(XI)

mit einem zur Chlorierung geeigneten Säurechlorid, wie z.B. Thionylchlorid oder Phthalsäuredichlorid, bei Temperaturen zwischen 0° C und 100° C umsetzt und das Produkt durch Vakuumdestillation isoliert.

Man erhält die neue Verbindung der Formel (XI) wenn man 2-Chlor-1-methyl-2,3,3-trifluor-cyclobutan-1-carbonsäureester der Formel (XII)

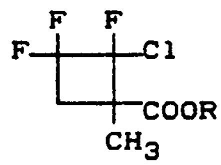

(XII)

in welcher

R für Niederalkyl, vorzugsweise für $C_1$-$C_4$-Alkyl, insbesondere für Methyl oder Ethyl steht,

mit einer wäßrigen Lösung eines Alkalimetallhydroxids, wie z.B. mit wäßriger Natronlauge, bei Temperaturen zwischen 50° C und 100° C umsetzt, dann mit einer starken Säure, wie z.B. Salzsäure, ansäuert, mit einem mit Wasser praktisch nicht mischbaren organischen Lösungsmittel, wie z.B. Methylenchlorid, extrahiert und das Produkt aus der organischen Phase durch Vakuumdestillation isoliert.

Man erhält die neuen Verbindungen der Formel (XII) wenn man Methacrylsäureester der Formel (XIII)

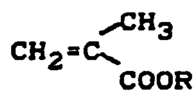

(XIII)

in welcher

R für Niederalkyl, vorzugsweise mit 1 bis 4 Kohlenstoffatomen, insbesondere für Methyl oder Ethyl steht,

mit Chlortrifluorethan, vorzugsweise in Gegenwart eines Stabilisators, wie z.B. Hydrochinon, in einem Autoklaven auf Temperaturen zwischen 50° C und 150° C erhitzt und anschließend das Produkt durch Vakuumdestillation isoliert.

Die neue Ausgangsverbindung der Formel (VIa)

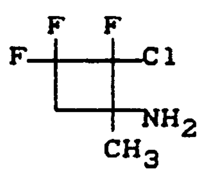

(VIa)

erhält man, wenn man 2-Chlor-1-methyl-2,3,3-trifluorcyclobutan-1-carbonsäureamid der Formel (XIV)

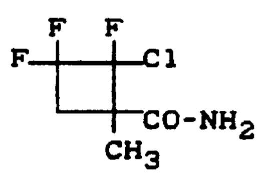

(XIV)

mit Natriumhypochlorit in Wasser bei Temperaturen zwischen 0° C und 20° C umsetzt, anschließend mit einer starken Base, wie z.B. Natriumhydroxid, behandelt, auf eine Temperatur zwischen 60° C und 100° C erwärmt und schließlich eine Grobreinigung durch Wasserdampfdestillation durchführt. Das als Wasserdampfdestillat angefallene ölige Produkt kann durch Vakuumdestilation weiter gereinigt werden.

Das als Zwischenprodukt benötigte 2-Chlor-1-methyl-2,3,3-trifluor-cyclobutan-1-carbonsäureamid der Formel (XIV) ist noch nicht aus der Literatur bekannt.

Man erhält die neue Verbindung der Formel (XIV), wenn man 2-Chlor-1-methyl-2,3,3-trifluor-cyclobutan-1-carbonsäureester der Formel (XII)

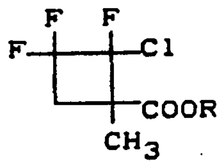

(XII)

mit Ammoniak, gegebenenfalls in Gegenwart eines Verdünnungsmittels, wie z.B. Methanol, und gegebenenfalls in Gegenwart eines Reaktionshilfsmittels, wie z.B. Natriummethylat, im Autoklaven bei Temperaturen zwischen 20°C und 80°C umsetzt, anschließend unter Eiskühlung ansäuert, mit einem mit Wasser praktisch nich mischbaren organischen Lösungsmittel, wie z.B. Methylenchlorid, extrahiert und aus der organischen Phase das Produkt durch Vakuumdestillation isoliert.

Die neuen Verbindungen der Formeln (IVa), (VIa), (X), (XI), (XII) und (XIV) lassen sich in einer gemeinsamen Formel (XV)

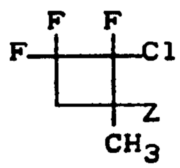

(XV)

in welcher

Z für N=C=O, $NH_2$, oder CO-Cl, CO-OH, CO-OR,CO-$NH_2$ steht, wobei

R für Niederalkyl steht,

zusammenfassen.

Als Säuren zur Durchführung des erfindungsgemäßen Verfahrens (a) kommen alle üblicherweise für Hydrazonspaltungen verwendbaren anorganischen und organischen Säuren infrage. Vorzugsweise verwendet man anorganische Mineralsäuren wie Chlorwasserstoffsäure, Schwefelsäure oder Phosphorsäure.

Als Verdünnungsmittel zur Durchführung des erfindungsgemäßen Verfahrens (a) kommen alle üblichen organischen oder anorganischen Lösungsmittel infrage. Vorzugsweise verwendet man polare mit Wasser mischbare organische Lösungsmittel, insbesondere Alkohole, wie Methanol Ethanol, Propanol oder Butanol, deren Gemische mit Wasser oder reines Wasser als Verdünnungsmittel.

Die Reaktionstemperaturen können bei der Durchführung des erfindungsgemäßen Verfahrens (a) in einem größeren Bereich variiert werden. Im allgemeinen arbeitet man bei Temperaturen zwischen 20°C und 150°C, vorzugsweise bei Temperaturen zwischen 50°C und 120°C.

Das erfindungsgemäße Verfahren (a) wird üblicherweise bei Normaldruck oder unter vermindertem Druck durchgeführt. Arbeitet man unter vermindertem Druck, so kommen Druckbereiche zwischen 20 und 400 mbar, vorzugsweise zwischen 100 und 200 mbar infrage.

Zur Durchführung des erfindungsgemäßen Verfahrens (a) setzt man pro Mol an Hydrazon der Formel (II) im allgemeinen 0.01 bis 50 Mol, vorzugsweise 0.01 bis 20 Mol an Säure ein. Dabei löst man das Hydrazon der Formel (II) in einer geeigneten Menge an Verdünnungsmittel, setzt dann die erforderliche Menge Säure zu und engt die Mischung unter vermindertem Druck über mehrere Stunden langsam ein.

In einer besonderen Durchführungsform ist es auch möglich, das erfindungsgemäße Verfahren (a) und die Herstellung der dafür erforderlichen Vorprodukte der Formel (II) in einem Reaktionsschritt in einem sogenannten Eintopfverfahren durchzuführen.

Dabei gibt es die Möglichkeit, als Ausgangsverbindungen die Triazolone der Formel (X) zu wählen und diese nacheinander im Eintopfverfahren mit Aminen der Formel (VI) und anschließend mit Säure gemäß dem erfindungsgemäßen Verfahren (a) umzusetzen, oder als Ausgangsverbindungen die Triazolon-Hydrazone der Formel (VIII) zu wählen und diese nacheinander im Eintopfverfahren mit Chlorameisensäureestern der Formel (IX), dann mit Aminen der Formel (VI) und anschließend mit Säure gemäß dem erfindungsgemäßen Verfahren (a) umzusetzen, oder als Ausgangsverbindungen die Triazolone der Formel (III) zu wählen und diese nacheinander im Eintopfverfahren mit Aldehyden oder Ketonen der Formel (VII), dann mit Isocyanaten der Formel (IV) und anschließend mit Säure gemäß dem erfindungsgemäßen Verfahren (a) umzusetzen.

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gege-

benenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester.

Das erfindungsgemäße Verfahren (b) wird gegebenenfalls in Gegenwart eines geeigneten Reaktionshilfsmittels durchgeführt. Als solche kommen alle üblichen anorganischen oder organischen Basen in Frage. Hierzu gehören beispielsweise tertiäre Amine, wie Triethylamin, N,N-Dimethylanilin, N,N-Diethylbenzylamin, N,N-Dimethylcyclohexylamin oder Dibutylzinndilaureat, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 150 °C, vorzugsweise bei Temperaturen zwischen 20 °C und 100 °C.

Zur Durchführung des erfindungsgemäßen Verfahrens (b) setzt man pro Mol an Triazolon der Formel (III) im allgemeinen 1.0 bis 2.0 Mol, vorzugsweise 1.0 bis 1.5 Mol an Isocyanat der Formel (IV) und gegebenenfalls 0.001 bis 2.0 Mol, vorzugsweise 0.001 bis 1.0 Mol an Reaktionshilfsmittel ein.

Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphor säuretriamid oder Ester, wie Essigsäureethylester, oder Sulfoxide, wie Dimethylsulfoxid.

Das erfindungsgemäße Verfahren (c) kann gegebenenfalls in Gegenwart eines geeigneten Reaktionshilfsmittels durchgeführt werden. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 120 °C, vorzugsweise bei Temperaturen zwischen 20 °C und 50 °C.

Zur Durchführung des erfindungsgemäßen Verfahrens (c) setzt man pro Mol an Oxycarbonyltriazolon der Formel (V) im allgemeinen 1.0 bis 5.0 Mol, vorzugsweise 1.0 bis 2.5 Mol an Amin der Formel (VI) und gegebenenfalls 1.0 bis 2.0 Mol, vorzugsweise 1.0 bis 1.2 Mol an Reaktionshilfsmittel ein.
Die Reaktionsdurchführung, Aufarbeitung und Isolierung der Reaktionsprodukte erfolgt nach allgemein üblichen Methoden.

In einer besonderen Durchführungsform ist es auch möglich, das erfindungsgemäße Verfahren (c) und die Herstellung der dafür erforderlichen Vorprodukte der Formel (V) in einem Reaktionsschritt in einem sogenannten Eintopfverfahren durchzuführen.

Man geht dabei von Triazolonen der Formel (III) aus und setzt diese nacheinander im Eintopfverfahren zunächst mit Chlorameisensäureestern der Formel (IX) und anschließend mit Aminen der Formel (VI) gemäß dem erfindungsgemäßen Verfahren (c) um.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria,

Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich auch zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffe-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischen Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierung enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyliden]cyclohexancarbonsäure (ALLOXYDIM); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopr opylamino)-1,3,5-triazin (CYANAZIN); 2-[4-(2,4-Dichlorphenoxy)-phenoxy]-propionsäure, deren Methyl-oder deren Ethylester (DICLOFOP); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl-oder deren Ethylester (FENOXAPROP); 2-[4-(5-Trifluormethyl-2-pyridyloxy)-phenoxy]-propansäure oder deren Butylester (FLUAZIFOP); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[(3-Chlor-5-(trifluormethyl)-2-pyridinyl)-oxy]-phenoxy}-propansäure bzw. deren Ethylester (HALOXYFOP); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 4-(Di-n-propylamino)-3,5-dinitrobenzolsulfonamid (ORYZALIN); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); o-(6-Chlor-3-phenyl-pyridazin-4-yl)-S-octyl-thiocarbonat (PYRIDATE); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-silfonyl]-thiophen-2-carbonsäure-methylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN); und 2-[4-(6-Chlor-2-chinoxalinyloxy)-phenoxy]-propionsäure-ethylester (QUIZALOFOP). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1:

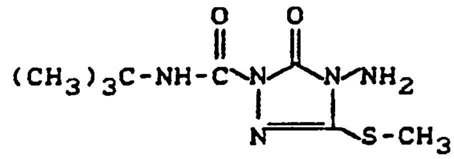

(Verfahren(a)-Eintopfvariante)

Eine Mischung aus 7,3 g (0,05 Mol) 4-Amino-5-methylthio-2,4-dihydro-3H-1,2,4-triazol-3-on, einer Spatelspitze p-Toluolsulfonsäure und 100 ml Aceton wird 3 Stunden unter Rückfluß erhitzt. Nach Einengen wird der Rückstand in 50 ml Acetonitril aufgenommen und nach Zugabe von 5,5 g (0,055 Mol) tert-Butylisocyanat und 0,1 g Diazabicycloundecen (DBU) wird das Gemisch 12 Stunden bei 20°C gerührt.

Nach erneutem Einengen wird der verbleibende Rückstand in 100 ml Ethanol/Wasser (1:1) aufgenommen und nach Zugabe von 5 ml konz. Salzsäure bei 60°C und leichtem Unterdruck innerhalb von 3 Stunden zur Trockne eingeengt. Der Rückstand wird in Methylenchlorid aufgenommen, mit Wasser neutral gewaschen, mit Natriumsulfat getrocknet und filtriert. Vom Filtrat wird das Lösungsmittel im Wasserstrahlvakuum abdestilliert und das im Rückstand verbleibende Produkt durch Verreiben mit Diethylether zur Kristallisation gebracht. Man erhält 6,0 g (49% der Theorie) 4-Amino-5-methylthio-2-tert-butylaminocarbonyl-2,4-dihydro-3H-1,2,4-triazol-3-on vom Schmelzpunkt 134°C.

Beispiel 2:

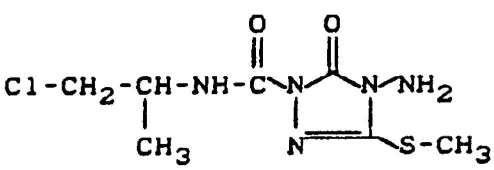

(Verfahren (b))

7,3 g (0,05 Mol) 4-Amino-5-methylthio-2,4-dihydro-3H-1,2,4-triazol-3-on werden in 100 ml Acetonitril suspendiert, mit 1 g Diazabicycloundecen (DBU) und mit 6,0 g (0,05 Mol) 2-Chlor-1-methyl-ethylisocyanat unter Rühren versetzt und 12 Stunden bei 20°C gerührt.

Dann wird eingeengt, der Rückstand in Methylenchlorid aufgenommen, neutral gewaschen, mit Natriumsulfat getrocknet und filtriert. Vom Filtrat wird das Lösungsmittel im Wasserstrahlvakuum abdestilliert und das im Rückstand verbleibende Produkt durch Verreiben mit Diethylether zur Kristallisation gebracht. Man erhält 6,0 g (45% der Theorie) 4-Amino-5-methylthio-2-(2-chlor-1-methyl-ethylaminocarbonyl)-2,4-dihydro-3H-1,2,4-triazol-3-on vom Schmelzpunkt 136°C.

Analog zu den Beispielen 1 und 2 und entsprechend der allgemeinen Beschreibung der erfindungsgemäßen Herstellungsverfahren können die in der nachstehenden Tabelle 1 aufgeführten Verbindungen der Formel (I) hergestellt werden.

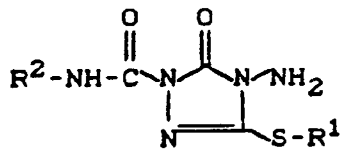

(I)

Tabelle 1: Beispiele für die Verbindungen der Formel I

| Bsp. Nr. | $R^1$ | $R^2$ | Schmelzpunkt (°C) |
|---|---|---|---|
| 3 | $CH_3$ | 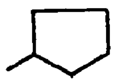 | 110 |
| 4 | $CH_3$ | $-C(CH_3)_2C\equiv CH$ | 178 |
| 5 | $CH_3$ | $-C(CH_3)_2CH_2Cl$ | 125 |
| 6 | $CH_3$ | $-C(CH_3)_2(CH_2)_3CH_3$ | 95 |
| 7 | $CH_3$ | 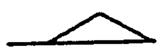 | 124 |
| 8 | $CH_3$ | $-C(CH_3)_2CHCl_2$ | 172 |
| 9 | $CH_3$ | $-C(CH_2Cl)_2$<br>$CH_3$ | 141 |
| 10 | $CH_3$ | $-C(CH_3)_2CH_2F$ | 152 |
| 11 | $CH_3$ | $-C(CH_3)_2CN$ | 153 |
| 12 | $C_2H_5$ | $-C(CH_3)_3$ | |
| 13 | $C_2H_5$ | $-C(CH_3)_2CH_2Cl$ | 101 |
| 14 | $C_2H_5$ | $-C(CH_3)_2CH_2F$ | |
| 15 | $CH_3$ | $-CH_2-CF_3$ | |
| 16 | $CH_3$ | $-CH(CH_3)(CF_3)$ | |
| 17 | $CH_3$ | $-CH(C_2H_5)CH_2Cl$ | |
| 18 | $CH_3$ | $-CH(CH_3)CHCl(CH_3)$ | |
| 19 | $CH_3$ | $-CH(CH_2Cl)CH=CH_2$ | |

Tabelle 1 - Fortsetzung

| Bsp.-Nr. | $R^1$ | $R^2$ | Schmelzpunkt (°C) |
|---|---|---|---|
| 20 | $CH_3$ | $-CH(CH_3)-CH=CCl_2$ | |
| 21 | $CH_3$ | $-C(CH_3)(CH_2F)_2$ | |
| 22 | $CH_3$ | $-C(CH_3)_2CF_3$ | |
| 23 | $CH_3$ | $-CH(CH_3)C_3H_7$-n | |
| 24 | $CH_3$ | $-CH(CH_3)C_3H_7$-iso | |
| 25 | $CH_3$ | $-C(CH_3)(CN)C_3H_7$-iso | |
| 26 | $CH_3$ | $-CH(C_2H_5)_2$ | |
| 27 | $CH_3$ | $-C(CH_3)_2C_2H_5$ | |
| 28 | $CH_3$ | $-C(CH_3)_2CH=CCl_2$ | |
| 29 | $CH_3$ | $-C_6H_{13}$-n | |
| 30 | $CH_3$ | $-CH(CH_3)CH_2C_3H_7$-iso | |
| 31 | $CH_3$ | $-CH(CH_3)C_4H_9$-tert. | |
| 32 | $CH_3$ | $-C(CH_3)_2C_3H_7$-n | |
| 33 | $CH_3$ | $-C(CH_3)_2C_3H_7$-i | |
| 34 | $CH_3$ | $-C(CH_3)(C_2H_5)_2$ | |
| 35 | $CH_3$ | $-CH(CH_3)C_5H_{11}$-n | |
| 36 | $CH_3$ | $-CH(CH_3)C_4H_9$-n | |
| 37 | $CH_3$ | $-CH(CH_3)CH_2CH_2-C_3H_7$-iso | |
| 38 | $CH_3$ | $-CH(CH_3)CH_2-C_4H_9$-tert. | |
| 39 | $CH_3$ | $-C(CH_3)_2CH(CH_3)CH=CCl_2$ | |

Tabelle 1 - Fortsetzung

| Bsp.-Nr. | $R^1$ | $R^2$ | Schmelzpunkt (°C) |
|---|---|---|---|
| 40 | $CH_3$ | $-C(C_2H_5)_3$ | |
| 41 | $CH_3$ | $-CH(CH_3)C_6H_{13}$-n | |
| 42 | $CH_3$ | $-C(CH_3)_2-CH_2-C_4H_9$-t | |
| 43 | $CH_3$ | $-CH(CH_3)-(CH_2)_3-C_3H_7$-iso | |
| 44 | $CH_3$ | 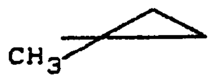 | |
| 45 | $CH_3$ | 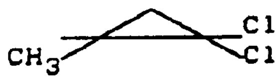 | |
| 46 | $CH_3$ | 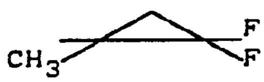 | |
| 47 | $CH_3$ | 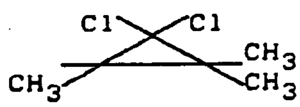 | |
| 48 | $CH_3$ | 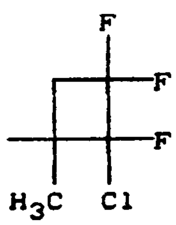 | 140 |
| 49 | $CH_3$ | 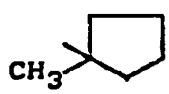 | |
| 50 | $CH_3$ | 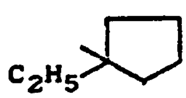 | |
| 51 | $CH_3$ | 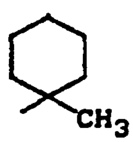 | |

Tabelle 1 - Fortsetzung

| Bsp.-Nr. | $R^1$ | $R^2$ | Schmelzpunkt (°C) |
|---|---|---|---|
| 52 | $CH_3$ | 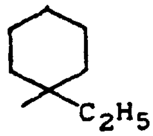 | |
| 53 | $CH_3$ | 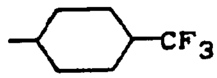 | |
| 54 | $CH_3$ | 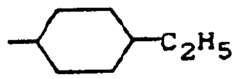 | |
| 55 | $CH_3$ | 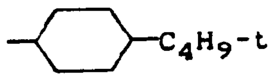 | |
| 56 | $CH_3$ | 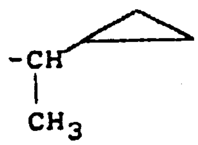 | |
| 57 | $CH_3$ | 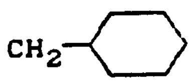 | |
| 58 | $CH_3$ | 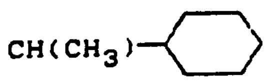 | |
| 59 | $CH_3$ | 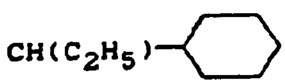 | |
| 60 | $CH_3$ | 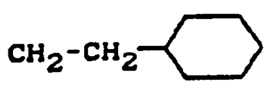 | |
| 61 | $CH_3$ | 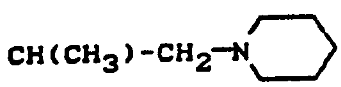 | |

<u>Tabelle 1</u> - Fortsetzung

| Bsp.-Nr. | $R^1$ | $R^2$ | Schmelzpunkt (°C) |
|---|---|---|---|
| 62 | $CH_3$ | $CH(CH_3)-CH_2-$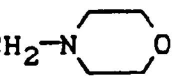 | |
| 63 | $CH_3$ | $C(CH_3)_2-CH_2-$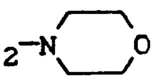 | |
| 64 | $CH_3$ | $CH(CH_3)-CH_2-OCH_3$ | |
| 65 | $CH_3$ | $CH(CH_3)CH_2-O-C_6H_5$ | |
| 66 | $CH_3$ | 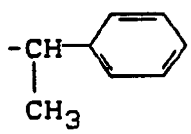 | |
| 67 | $CH_3$ | 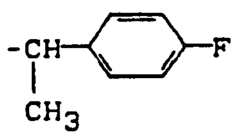 | 171 |
| 68 | $CH_3$ | 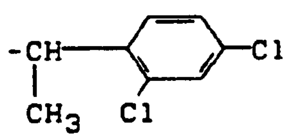 | 230 |
| 69 | $CH_3$ | 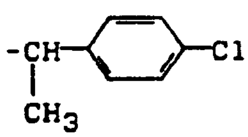 | 147 |
| 70 | $CH_3$ | 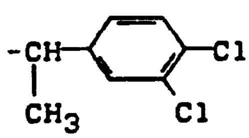 | 132 |
| 71 | $CH_3$ | 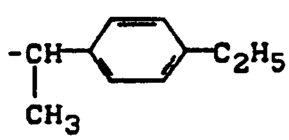 | 105 |

Tabelle 1 - Fortsetzung

| Bsp.-Nr. | $R^1$ | $R^2$ | Schmelzpunkt (°C) |
|---|---|---|---|
| 72 | $CH_3$ | 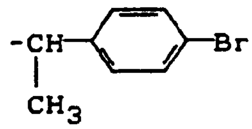 | 122 |
| 73 | $CH_3$ | 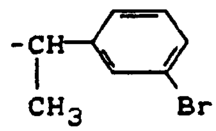 | 117 |
| 74 | $CH_3$ | 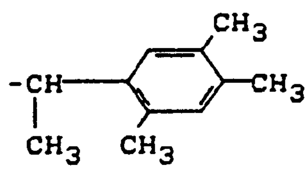 | 127 |
| 75 | $CH_3$ | 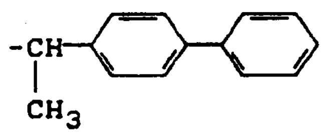 | 167 |
| 76 | $n-C_3H_7$ | $C(CH_3)_2CH_2Cl$ | |
| 77 | $CH_3$ | $-\underset{\displaystyle CH_3}{CH}-C_2H_5$ | 102 |
| 78 | $CH_2CH=CH_2$ | $-C(CH_3)_2CH_2Cl$ | 97 |
| 79 | $CH_3$ | $-(CH_2)_2-O-C_2H_5$ | 163 |

Die in Tabelle 1 als Beispiel 70 aufgeführte Verbindung kann beispielsweise wie nachstehend beschrieben hergestellt werden:

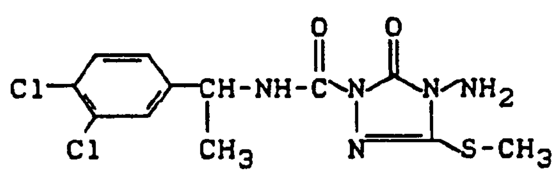

(Verfahren (c))

Eine Mischung aus 12,6 g (0.05 Mol) 4-Amino-5-methyl-thio-2-phenoxycarbonyl-2,4-dihydro-3H-1,2,4-triazol-3-on, 9,5 g (0,05 Mol) 1-(3,4-Dichlor-phenyl)-ethylamin und 100 ml Tetrahydrofuran wird 16 Stunden bei

20°C gerührt und anschließend eingeengt. Der Rückstand wird mit Essigsäureethylester verrieben und das kristallin angefallene Produkt durch Absaugen isoliert.

Man erhält 2,9 g (16% der Theorie) 4-Amino-5-methylthio-2-(1-(3,4-dichlor-phenyl)-ethylaminocarbonyl)-2,4-dihydro-3H-1,2,4-triazol-3-on vom Schmelzpunkt 132°C.

Ausgangstoffe der Formel (V):

Beispiel (V-1)

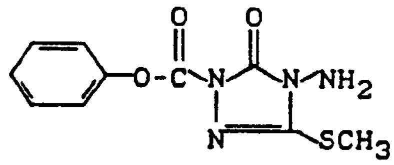

Eine Mischung aus 201 g (1,40 Mol) 4-Amino-5-methylthio-2,4-dihydro-3H-1,2,4-triazol-3-on und 1000 ml Tetrahydrofuran wird bei 0° C portionsweise mit 170 g (1,5 Mol) Kalium-tert.-butylat versetzt. Das Reaktionsgemisch wird eine weitere Stunde bei 0° C gerührt. Anschließend werden unter Rühren bei 0°C 214 g (1,4 Mol) Chlorameisensäurephenylester zugetropft. Die Reaktionslösung wird weitere 15 Stunden bei 20°C gerührt und am Rotationsverdampfer eingeengt. Der Rückstand wird mit 300 ml Wasser und 500 ml Methylenchlorid versetzt. An der Phasengrenze scheidet sich ein farblose Feststoff ab, der durch Absaugen isoliert wird.

Man erhält 218 g (63% der Theorie) 4-Amino-5-methylthio-2-phenoxycarbonyl-2,4-dihydro-3H-1,2,4-triazol-3-on vom Schmelzpunkt 161°C.

Analog erhält man

Beispiel (V-2)

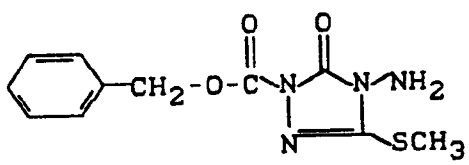

4-Amino-5-methylthio-2-benzyloxycarbonyl-2,4-dihydro-3H-1,2,4-triazol-3-on.

Beispiel IVa

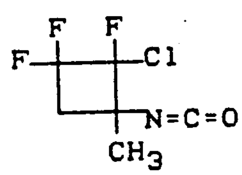

100 g (0,45 Mol) 1-Methyl-2-chlor-2,3,3-trifluorcyclobutancarbonsäurechlorid werden zu einer Lösung aus 61,3 g (0,53 Mol) Trimethylsilylazid in 250 ml trockenem Benzol getropft. Es wird auf 75°C erwärmt und bis zum Ende der Gasentwicklung gerührt. Anschließend wird das Lösungsmittel abdestilliert und das zurückbleibende Produkt fraktioniert.

Man erhält 77 g (86 % der Theorie) des o.g. Produktes, Siedepunkt: 75-78°C/80 mm Hg.

Beispiel X

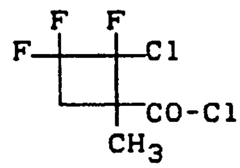

220 g (1,09 Mol) 1-Methyl-2-chlor-2,3,3-trifluorcyclobutancarbonsäure werden mit 250 g (1,23 Mol) Phthalsäuredichlorid über Nacht bei Raumtemperatur gerührt. Anschließend wird das Säurechlorid abdestilliert.

Man erhält 238 g (99 % der Theorie) des o.g. Produktes, Siedepunkt: 54-56°C/23 mm Hg.

Beispiel XI

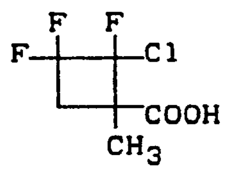

573 g (2,65 Mol) 1-Methyl-2-chlor-2,3,3-trifluorcyclobutancarbonsäuremethylester, 233 g (5,8 Mol) Natriumhydroxid und 1000 ml Wasser werden drei Stunden bei 80° C gerührt. Es wird mit konz. Salzsäure angesäuert, mit Dichlormethan extrahiert, die organischen Phasen getrocknet und destilliert. Man erhält 472 g (88 % der Theorie) des oben genannten Produkts, Siedepunkt: 112-116°C/16 mm Hg (60 % trans-Isomeres, 40 % cis-Isomeres).

Beispiel XII-1

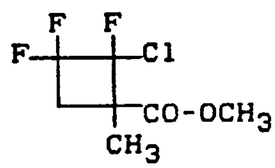

750 g (7,5 Mol) Methacrylsäuremethylester, 700 g (6,0 Mol) Chlortrifluorethen und 3 g Hydrochinon werden in einem Stahlautoklaven 12 Stunden auf 120°C erhitzt. Das Produkt wird direkt fraktioniert.

Man erhält 780 g (60% der Theorie des oben genannten Produktes, Siedepunkt: 57-59°C/14 mm Hg.

Beispiel XIV

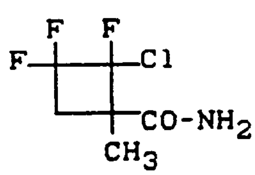

400 g (1,8 Mol) 1-methyl-2-chlor-2,3,3-trifluorcyclobutancarbonsäuremethylester und 62 ml 30 %ige Natriummethylat-Lösung werden in 1500 ml Methanol gelöst und im Autoklaven mit 620 ml flüssigen Ammoniak versetzt. Es wird 14 Stunden bei 50°C gerührt, auf Eis gegossen, mit Salzsäure schwach angesäuert und mit Dichlormethan extrahiert. Die vereinigten organischen Phasen werden mit verdünnter Hydrogencarbonat-Lösung gewaschen, getrocknet und eingedempft. Das Amid wird destilliert.

Man erhält 254 g (68 % der Theorie) des o.g. Produktes, Siedepunkt: 138-140°C/20 mm Hg.

Beispiel VIa

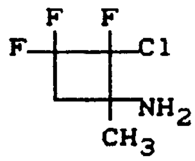

Zu einer Suspension aus 200 g (0,99 Mol) 1-methyl-2-chlor-2,3,3-trifluorcyclobutancarbonsäureamid in 1,2 l Wasser werden bei 10°C 571 ml 13 %ige Natriumhypochlorit-Lösung (= 1 mol NaOCl) getropft, wobei sich das Amid langsam auflöst. Es wird noch eine Stunde bei 10°C gerührt und 400 ml 50 %ige NaOH zugetropft. Anschließend wird auf 80°C erwärmt, eine Stunde bei 80°C gerührt und wasserdampfdestilliert. Das Amin wird abgetrennt und fraktioniert.

Man erhält 105 g (61 % der Theorie) des o.g. Produktes, Siedepunkt 54-55°C/48 mm Hg.

Anwendungsbeispiele:

In den folgenden Anwendungsbeispielen wurden die nachstehend aufgeführten Verbindungen als Vergleichssubstanzen herangezogen:

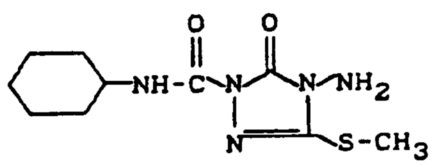

(A)

4-Amino-5-methylthio-2-cyclohexylaminocarbonyl-2,4-dihydro-3H-1,2,4-triazol-3-on

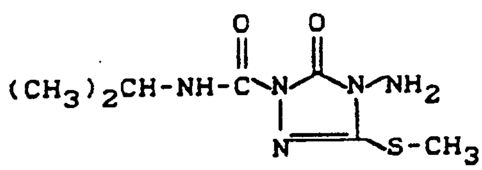

(B)

4-Amino-5-methylthio-2-isopropylaminocarbonyl-2,4-dihydro-3H-1,2,4-triazol-3-on

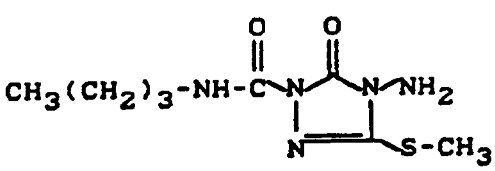

(C)

4-Amino-5-methylthio-2-butylaminocarbonyl-2,4-dihydro-3H-1,2,4-triazol-3-on

(alle bekannt aus JP 52-125168).

Beispiel A

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der

angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegenüber den bekannten Verbindungen (A) und (C) zeigen in diesem Test z.B. die Verbindungen gemäß den Herstellungsbeispielen (1), (4), (5), (9) und (10).

Beispeil B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit (bei guter Nutzpflanzenselektivität) gegenüber den bekannten Verbindungen (A), (B) und (C) zeigen in diesem Test z.B. die Verbindungen gemäß den Herstellungsbeispielen (3), (4), (5), (6), (7), (10), (67), (71) und (73).

## Patentansprüche

1. Substituierte 4-Amino-5-alkylthio-1,2,4-triazol-3-one der Formel (I)

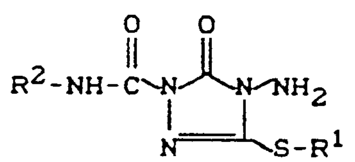

(I)

in welcher

$R^1$ für jeweils geradkettiges oder verzweigtes Alkyl, Alkenyl oder Alkinyl mit jeweils bis zu 4 Kohlenstoffatomen steht und

$R^2$ für sec.-Butyl, tert.-Butyl, jeweils geradkettiges oder verzweigtes $C_5$-$C_{10}$-Alkyl, $C_5$-$C_{10}$-Alkenyl oder $C_5$-$C_{10}$-Alkinyl, für jeweils durch Halogen, Cyano, $C_3$-$C_6$-Cycloalkyl, Aryloxy mit 6 bis 10 Kohlenstoffatomen oder $C_1$-$C_6$-Alkoxy substituiertes, geradkettiges oder verzweigtes Alkyl, Alkenyl oder Alkinyl mit jeweils bis zu 10 Kohlenstoffatomen, für jeweils durch Piperidyl oder Morpholinyl substituiertes $C_1$-$C_6$-Alkyl, für gegebenenfalls durch Halogen, Aryl mit 6 bis 10 Kohlenstoffatomen, $C_1$-$C_6$-Alkyl oder $C_1$-$C_4$-Halogenalkyl substituiertes Cyclopropyl, Cyclobutyl, Cyclopentyl oder Cycloheptyl, für durch Halogen, $C_1$-$C_6$-Alkyl, $C_2$-$C_4$-Alkenyl oder $C_1$-$C_4$-Halogenalkyl substituiertes Cyclohexyl, für jeweils gegebenenfalls in der aromatischen Komponente durch Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_4$-Alkylsulfinyl, $C_1$-$C_4$-Alkylsulfonyl, Phenoxy und/oder Phenyl substituiertes Phenyl-$C_1$-$C_2$-alkyl oder Naphthyl-$C_1$-$C_2$-alkyl steht.

2. Substituierte 4-Amino-5-alkylthio-1,2,4-triazol-3-one der Formel (I) gemäß Anspruch 1, in welcher
$R^1$ für Methyl, Ethyl, Propyl, Isopropyl, Butyl, Isobutyl, sec.-Butyl, tert.-Butyl, Allyl, Crotonyl oder Propargyl steht und
$R^2$ für sec.-Butyl, tert.-Butyl, jeweils geradkettiges oder verzweigtes Pentyl, Hexyl, Heptyl, Octyl, Pentenyl, Hexenyl, Heptenyl, Octenyl, Pentinyl, Hexinyl, Heptinyl oder Octinyl, für jeweils durch Fluor, Chlor, Brom, Cyano, Cyclopropyl, Cyclobutyl, Cyclopentyl, Cyclohexyl, Phen-oxy, Methoxy, Ethoxy, Propoxy, Isopropoxy, Butoxy, Isobutoxy, sec.-Butoxy oder tert.-Butoxy substituiertes Methyl, Ethyl, Propyl, Isopropyl, Butyl, Isobutyl, sec.-Butyl, tert.-Butyl, Pentyl, Isopentyl, sec.-Pentyl, tert.-Pentyl, Allyl, Crotonyl oder Propargyl steht, oder $R^2$ weiterhin für jeweils durch Piperidinyl oder Morpholinyl substituiertes Methyl, Ethyl, n- oder iso-Propyl, für gegebenenfalls jeweils durch Fluor, Chlor, Brom, Methyl, Trifluormethyl, Ethyl, Propyl, Isopropyl, Butyl, Isobutyl, sec.-Butyl oder tert.-Butyl substituiertes Cyclopropyl, Cyclobutyl oder Cyclopentyl, für durch Fluor, Chlor, Methyl, Trifluormethyl, Ethyl, Propyl, Isopropyl, Butyl, Isobutyl, sec.-Butyl, tert.-Butyl oder Propargyl substituiertes Cyclohexyl steht, $R^2$ ferner für jeweils gegebenenfalls in der aromatischen Komponente durch Fluor, Chlor, Brom, Methyl, Ethyl, Propyl, Isopropyl, Trifluormethyl, Methoxy, Ethoxy, Difluormethoxy, Trifluormethoxy, Methylthio, Methylsulfinyl, Methylsulfonyl, Phenoxy und/oder Phenyl substituiertes Benzyl oder Phenylethyl steht.

3. Verfahren zur Herstellung von substituierten 4-Amino-5-alkylthio-1,2,4-triazol-3-onen der Formel (I)

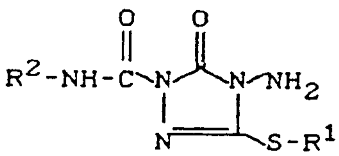

(I)

in welcher
$R^1$ für jeweils geradkettiges oder verzweigtes Alkyl, Alkenyl oder Alkinyl mit jeweils bis zu 4 Kohlenstoffatomen steht und
$R^2$ für sec.-Butyl, tert.-Butyl, jeweils geradkettiges oder verzweigtes $C_5$-$C_{10}$-Alkyl, $C_5$-$C_{10}$-Alkenyl oder $C_5$-$C_{10}$-Alkinyl, für jeweils durch Halogen, Cyano, $C_3$-$C_6$-Cycloalkyl, Aryloxy mit 6 bis 10 Kohlenstoffatomen oder $C_1$-$C_6$-Alkoxy substituiertes, geradkettiges oder verzweigtes Alkyl, Alkenyl oder Alkinyl mit jeweils bis zu 10 Kohlenstoffatomen, für jeweils durch Piperidyl oder Morpholinyl substituiertes $C_1$-$C_6$-Alkyl, für gegebenenfalls durch Halogen, Aryl mit 6 bis 10 Kohlenstoffatomen, $C_1$-$C_6$-Alkyl, oder $C_1$-$C_4$-Halogenalkyl substituiertes Cyclopropyl, Cyclobutyl, Cyclopentyl oder Cycloheptyl, für durch Halogen, $C_1$-$C_6$-Alkyl, $C_2$-$C_4$-Alkenyl oder $C_1$-$C_4$-Halogenalkyl substituiertes Cyclohexyl, für jeweils gegebenenfalls in der aromatischen Komponente durch Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_4$-Alkylsulfinyl, $C_1$-$C_4$-Alkylsulfonyl, Phenoxy, und/oder Phenyl, substituiertes Phenyl-$C_1$-$C_2$-alkyl oder Naphthyl-$C_1$-$C_2$-alkyl steht.
dadurch gekennzeichnet, daß man
(a) Hydrazone der allgemeinen Formel (II)

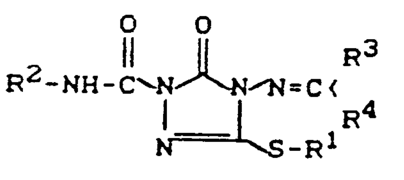

(II)

in welcher
$R^1$ und $R^2$ die oben angegebenen Bedeutungen haben und
$R^3$ und $R^4$ unabhängig voneinander jeweils für Wasserstoff, $C_1$-$C_6$-Alkyl, Phenyl-$C_1$-$C_4$-alkyl oder Phenyl stehen,
mit einer Säure, gegebenenfalls in Gegenwart eines Verdünnungsmittels umsetzt, oder daß man
(b) Triazolone der allgemeinen Formel (III)

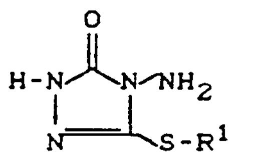

(III)

in welcher

$R^1$ die oben angegebene Bedeutung hat,

mit Isocyanaten der allgemeinen Formel (IV)

$R^2$ - N = C = O (IV)

in welcher

$R^2$die oben angegebene Bedeutung hat,

gegebenenfalls in Gegenwart eines Verdünnungsmittels und gegebenenfalls in Gegenwart eines Reaktionshilfsmittels umsetzt, oder daß man

(c) Oxycarbonyltriazolone der allgemeinen Formel (V)

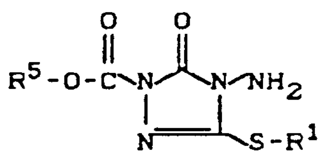

(V)

in welcher

$R^1$ die oben angegebene Bedeutung hat und

$R^5$ für $C_1$-$C_6$-Alkyl, Phenyl oder Phenyl-$C_1$-$C_4$-alkyl steht,

mit Aminen der allgemeinen Formel (VI)

$R^2$ - $NH_2$ (VI)

in welcher

$R^2$ die oben angegebene Bedeutung hat,

gegebenenfalls in Gegenwart eines Verdünnungsmittels und gegebenenfalls in Gegenwart eines Reaktionshilfsmittels umsetzt.

4. Herbizide Mittel, gekennzeichnet durch einen Gehalt an mindestens einem substituierten 4-Amino-5-alkylthio-1,2,4-trizaol-3-on der Formel (I) gemäß Anspruch 1.

5. Verfahren zur Bekämpfung von unerwünschten Pflanzen, dadurch gekennzeichnet, daß man substituierte 4-Amino-5-alkylthio-1,2,4-triazol-3-one der Formel (I) gemäß Anspruch 1 auf die unerwünschten Pflanzen und/oder ihren Lebensraum einwirken läßt.

6. Verwendung von substituierten 4-Amino-5-alkylthio-1,2,4-triazol-3-onen der Formel (I) gemäß Anspruch 1 zur Bekämpfung von unerwünschten Pflanzen.

7. Verfahren zur Herstellung von herbiziden Mitteln, dadurch gekennzeichnet, daß man substituierte 4-Amino-5-alkylthio-1,2,4-triazol-3-one der Formel (I) gemäß Anspruch 1 mit Streckmitteln und/oder oberflächenaktiven Substanzen vermischt.

8. Hydrazone der Formel (II)

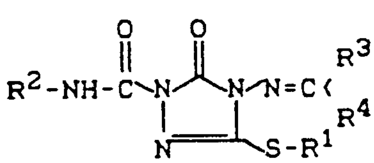

(II)

in welcher

$R^1$ für jeweils geradkettiges oder verzweigtes Alkyl, Alkenyl oder Alkinyl mit jeweils bis zu 4 Koh-

lenstoffatomen steht und

$R^2$ für sec.-Butyl, tert.-Butyl, jeweils geradkettiges oder verzweigtes $C_5$-$C_{10}$-Alkyl, $C_5$-$C_{10}$-Alkenyl oder $C_5$-$C_{10}$-Alkinyl, für jeweils durch halogen, Cyano, $C_3$-$C_6$-Cycloalkyl, Aryloxy mit 6 bis 10 Kohlenstoffatomen oder $C_1$-$C_6$-Alkoxy substituiertes, geradkettiges oder verzweigtes Alkyl, Alkenyl oder Alkinyl mit jeweils bis zu 10 Kohlenstoffatomen, für jeweils durch Piperidyl oder Morpholinyl substituiertes $C_1$-$C_6$-Alkyl, für gegebenenfalls durch Halogen, Aryl mit 6 bis 10 Kohlenstoffatomen, $C_1$-$C_6$-Alkyl oder $C_1$-$C_4$-Halogenalkyl substituiertes Cyclopropyl, Cyclobutyl, Cyclopentyl oder Cycloheptyl, für durch Halogen, $C_1$-$C_6$-Alkyl, $C_2$-$C_4$-Alkenyl oder $C_1$-$C_4$-Halogenalkyl substituiertes Cyclohexyl, für jeweils gegebenenfalls in der aromatischen Komponente durch Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_4$-Alkylsulfinyl, $C_1$-$C_4$-Alkylsulfonyl, Phenoxy und/oder Phenyl substituiertes Phenyl-$C_1$-$C_2$-alkyl oder Naphthyl-$C_1$-$C_2$-alkyl steht,

$R^3$ und $R^4$ unabhängig voneinander jeweils für Wasserstoff, $C_1$-$C_6$-Alkyl, Phenyl-$C_1$-$C_4$-alkyl oder Phenyl stehen.

**9.** Oxycarbonyltriazolone der Formel (V)

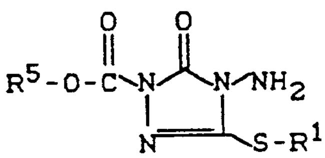

(V)

in welcher

$R^1$ für jeweils geradkettiges oder verzweigtes Alkyl, Alkenyl oder Alkinyl mit jeweils bis zu 4 Kohlenstoffatomen steht und

$R^5$ für $C_1$-$C_6$-Alkyl, Phenyl oder Phenyl-$C_1$-$C_4$-alkyl steht.

**10.** Triazolon-Hydrazone der Formel (VIII)

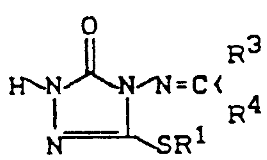

(VIII)

in welcher

$R^1$ für jeweils geradkettiges oder verzweigtes Alkyl, Alkenyl oder Alkinyl mit jeweils bis zu 4 Kohlenstoffatmen steht, und

$R^3$ und $R^4$ unabhängig voneinander jeweils für Wasserstoff, $C_1$-$C_6$-Alkyl, Phenyl-$C_1$-$C_4$-alkyl oder Phenyl stehen.

**11.** Triazolone der Formel (X)

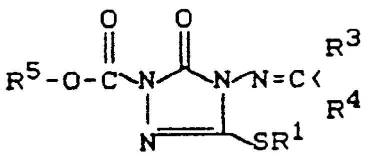

(X)

in welcher

$R^1$ für jeweils geradkettiges oder verzweigtes Alkyl, Alkenyl oder Alkinyl mit jeweils bis zu 4 Koh-

lenstoffatomen steht,

$R^3$ und $R^4$ unabhängig voneinander jeweils für Wasserstoff, $C_1$-$C_6$-Alkyl, Phenyl-$C_1$-$C_4$-alkyl oder Phenyl stehen und

$R^5$ für $C_1$-$C_6$-Alkyl, Phenyl oder Phenyl-$C_1$-$C_4$-alkyl steht.

**12.** Die Verbindung der Formel

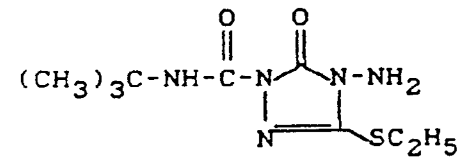

**13.** Die Verbindung der Formel

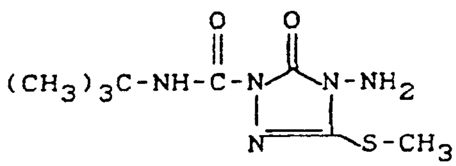

**14.** Die Verbindung der Formel

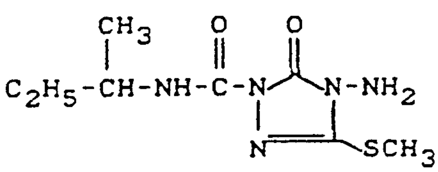

## Claims

**1.** Substituted 4-amino-5-alkylthio-1,2,4-triazol-3-ones of the formula (I)

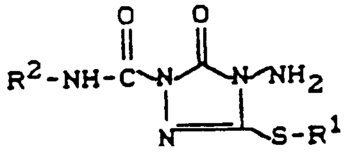

(I)

in which

$R^1$ represents in each case straight-chain or branched alkyl, alkenyl or alkinyl, each of which has up to 4 carbon atoms, and

$R^2$ represents sec.-butyl, tert.butyl, in each case straight-chain or branched $C_5$-$C_{10}$-alkyl, $C_5$-$C_{10}$-alkenyl or $C_5$-$C_{10}$-alkinyl, or represents straight-chain or branched alkyl, alkenyl or alkinyl, each of which has up to 10 carbon atoms, and each of which is substituted by halogen, cyano, $C_3$-$C_6$-cycloalkyl, aryloxy having 6 to 10 carbon atoms or $C_1$-$C_6$-alkoxy, or represents $C_1$-$C_6$-alkyl which is in each case substituted by piperidyl or morpholinyl, or represents cyclopropyl, cyclobutyl, cyclopentyl or cycloheptyl, each of which

is optionally substituted by halogen, aryl having 6 to 10 carbon atoms, $C_1$-$C_6$-alkyl or $C_1$-$C_4$-halogenoalkyl, or represents cyclohexyl which is substituted by halogen, $C_1$-$C_6$-alkyl, $C_2$-$C_4$-alkenyl or $C_1$-$C_4$-halogenoalkyl, or represents phenyl-$C_1$-$C_2$-alkyl or naphthyl-$C_1$-$C_2$-alkyl which is in each case optionally substituted in the aromatic component by halogen, $C_1$-$C_4$-alkyl, $C_1$-$C_2$-halogenoalkyl, $C_1$-$C_4$-alkoxy, $C_1$-$C_2$-halogenoalkoxy, $C_1$-$C_4$-alkylthio, $C_1$-$C_4$-alkylsulphinyl, $C_1$-$C_4$-alkylsulphonyl, phenoxy and/or phenyl.

**2.** Substituted 4-amino-5-alkylthio-1,2,4-triazol-3-ones of the formula (I) according to Claim 1, in which

$R^1$ represents methyl, ethyl, propyl, isopropyl, butyl, isobutyl, sec.-butyl, tert.-butyl, allyl, crotonyl or propargyl and

$R^2$ represents sec.-butyl, tert.-butyl, in each case straight-chain or branched pentyl, hexyl, heptyl, octyl, pentenyl, hexenyl, heptenyl, octenyl, pentinyl, hexinyl, heptinyl or octinyl, or represents methyl, ethyl, propyl, isopropyl, butyl, isobutyl, sec.-butyl, tert.-butyl, pentyl, isopentyl, sec.-pentyl, tert.-pentyl, allyl, crotonyl or propargyl, each of which is substituted by fluorine, chlorine, bromine, cyano, cyclopropyl, cyclobutyl, cyclopentyl, cyclohexyl, phenoxy, methoxy, ethoxy, propoxy, isopropoxy, butoxy, isobutoxy, sec.-butoxy or tert.-butoxy, or $R^2$ furthermore represents methyl, ethyl, n- or isopropyl, in each case substituted by piperidinyl or morpholinyl, or represents cyclopropyl, cyclobutyl or cyclopentyl, each of which is optionally substituted by fluorine, chlorine, bromine, methyl, trifluoromethyl, ethyl, propyl, isopropyl, butyl, isobutyl, sec.-butyl or tert.-butyl, or represents cyclohexyl which is substituted by fluorine, chlorine, methyl, trifluoromethyl, ethyl, propyl, isopropyl, butyl, isobutyl, sec.-butyl, tert.-butyl or propargyl, or $R^2$ further represents benzyl or phenylethyl which is in each case optionally substituted in the aromatic component by fluorine, chlorine, bromine, methyl, ethyl, propyl, isopropyl, trifluoromethyl, methoxy, ethoxy, difluoromethoxy, trifluoromethoxy, methylthio, methylsulphinyl, methylsulphonyl, phenoxy and/or phenyl.

**3.** Process for the preparation of substituted 4-amino-5-alkylthio-1,2,4-triazol-3-ones of the formula (I)

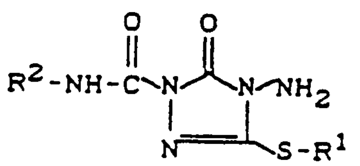

(I)

in which

$R^1$ represents in each case straight-chain or branched alkyl, alkenyl or alkinyl, each of which has up to 4 carbon atoms, and

$R^2$ represents sec.-butyl, tert.-butyl, in each case straight-chain or branched $C_5$-$C_{10}$-alkyl, $C_5$-$C_{10}$-alkenyl or $C_5$-$C_{10}$-alkinyl, or represents straight-chain or branched alkyl, alkenyl or alkinyl, each of which has up to 10 carbon atoms, and each of which is substituted by halogen, cyano, $C_3$-$C_6$-cycloalkyl, aryloxy having 6 to 10 carbon atoms or $C_1$-$C_6$-alkoxy, or represents $C_1$-$C_6$-alkyl which is in each case substituted by piperidyl or morpholinyl, or represents cyclopropyl, cyclobutyl, cyclopentyl or cycloheptyl, each of which is optionally substituted by halogen, aryl having 6 to 10 carbon atoms, $C_1$-$C_6$-alkyl or $C_1$-$C_4$-halogenoalkyl, or represents cyclohexyl which is substituted by halogen, $C_1$-$C_6$-alkyl, $C_2$-$C_4$-alkenyl or $C_1$-$C_4$-halogenoalkyl, or represents phenyl-$C_1$-$C_2$-alkyl or naphthyl-$C_1$-$C_2$-alkyl which is in each case optionally substituted in the aromatic component by halogen, $C_1$-$C_4$-alkyl, $C_1$-$C_2$-halogenoalkyl, $C_1$-$C_4$-alkoxy, $C_1$-$C_2$-halogenoalkoxy, $C_1$-$C_4$-alkylthio, $C_1$-$C_4$-alkylsulphinyl, $C_1$-$C_4$-alkylsulphonyl, phenoxy and/or phenyl.

characterized in that

(a) hydrazones of the general formula (II)

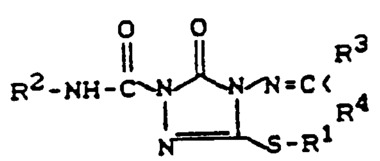

(II)

in which

$R^1$ and $R^2$ have the abovementioned meanings and

$R^3$ and $R^4$ independently of one another each represent hydrogen, $C_1$-$C_6$-alkyl, phenyl-$C_1$-$C_4$-alkyl or phenyl,

are reacted with an acid, if appropriate in the presence of a diluent,

or in that

(b) triazolones of the general formula (III)

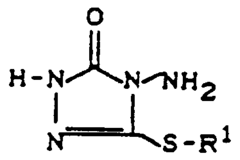

(III)

in which

$R^1$ has the abovementioned meaning

are reacted with isocyanates of the general formula (IV)

$$R^2 - N = C = O \quad (IV)$$

in which

$R^2$ has the abovementioned meaning,

if appropriate in the presence of a diluent and if appropriate in the presence of a reaction auxiliary,

or in that

(c) oxycarbonyltriazolones of the general formula (V)

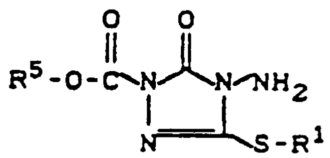

(V)

in which

$R^1$ has the abovementioned meaning and

$R^5$ represents $C_1$-$C_6$-alkyl, phenyl or phenyl-$C_1$-$C_4$-alkyl,

are reacted with amines of the general formula (VI)

$$R^2 - NH_2 \quad (VI)$$

in which

$R^2$ has the abovementioned meaning,

if appropriate in the presence of a diluent and if appropriate in the presence of a reaction auxiliary.

4. Herbicidal agents, characterized in that they contain at least one substituted 4-amino-5-alkylthio-1,2,4-triazol-3-one of the formula (I) according to Claim 1.

5. Method of combating unwanted plants, characterized in that substituted 4-amino-5-alkylthio-1,2,4-triazol-3-ones of the formula (I) according to Claim 1 are allowed to act on the unwanted plants and/or their environment.

6. Use of substituted 4-amino-5-alkylthio-1,2,4-triazol-3-ones of the formula (I) according to Claim 1 for combating weeds.

7. Process for the preparation of herbicidal agents, characterized in that substituted 4-amino-5-alkylthio-1,2,4-triazol-3-ones of the formula (I) according to Claim 1 are mixed with extenders and/or surface-active substances.

8. Hydrazones of the formula (II)

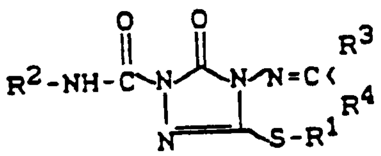

(II)

in which

$R^1$ represents in each case straight-chain or branched alkyl, alkenyl or alkinyl, each of which has up to 4 carbon atoms, and

$R^2$ represents sec.-butyl, tert.-butyl, in each case straight-chain or branched $C_5$-$C_{10}$-alkyl, $C_5$-$C_{10}$-alkenyl or $C_5$-$C_{10}$-alkinyl, or represents straight-chain or branched alkyl, alkenyl or alkinyl, each of which has up to 10 carbon atoms, and each of which is substituted by halogen, cyano, $C_3$-$C_6$-cycloalkyl, aryloxy having 6 to 10 carbon atoms or $C_1$-$C_6$-alkoxy, or represents $C_1$-$C_6$-alkyl which is in each case substituted by piperidyl or morpholinyl, or represents cyclopropyl, cyclobutyl, cyclopentyl or cycloheptyl, each of which is optionally substituted by halogen, aryl having 6 to 10 carbon atoms, $C_1$-$C_6$-alkyl or $C_1$-$C_4$-halogenoalkyl, or represents cyclohexyl which is substituted by halogen, $C_1$-$C_6$-alkyl, $C_2$-$C_4$-alkenyl or $C_1$-$C_4$-halogenoalkyl, or represents phenyl-$C_1$-$C_2$-alkyl or naphthyl-$C_1$-$C_2$-alkyl which is in each case optionally substituted in the aromatic component by halogen, $C_1$-$C_4$-alkyl, $C_1$-$C_2$-halogenoalkyl, $C_1$-$C_4$-alkoxy, $C_1$-$C_2$-halogenoalkoxy, $C_1$-$C_4$-alkylthio, $C_1$-$C_4$-alkylsulphinyl, $C_1$-$C_4$-alkylsulphonyl, phenoxy and/or phenyl, and

$R^3$ and $R^4$ independently of one another each represent hydrogen, $C_1$-$C_6$-alkyl, phenyl-$C_1$-$C_4$-alkyl or phenyl.

**9.** Oxycarbonyltriazolones of the formula (V)

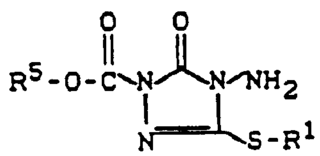

(V)

in which

$R^1$ represents in each case straight-chain or branched alkyl, alkenyl or alkinyl, each of which has up to 4 carbon atoms, and

$R^5$ represents $C_1$-$C_6$-alkyl, phenyl or phenyl-$C_1$-$C_4$-alkyl.

**10.** Triazolone-hydrazones of the formula (VIII)

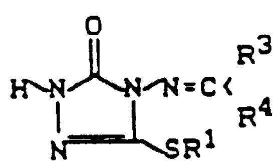

(VIII)

in which

$R^1$ represents in each case straight-chain or branched alkyl, alkenyl or alkinyl, each of which has up to 4 carbon atoms, and

$R^3$ and $R^4$ independently of one another each represent hydrogen, $C_1$-$C_6$-alkyl, phenyl-$C_1$-$C_4$-alkyl or phenyl.

**11.** Triazolones of the formula (X)

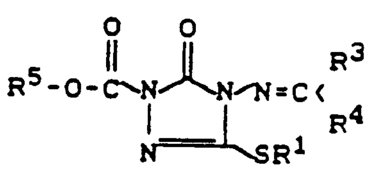

(X)

in which

$R^1$ represents in each case straight-chain or branched alkyl, alkenyl or alkinyl, each of which has up to 4 carbon atoms,

$R^3$ and $R^4$ independently of one another each represent hydrogen, $C_1$-$C_6$-alkyl, phenyl-$C_1$-$C_4$-alkyl or phenyl and

$R^5$ represents $C_1$-$C_6$-alkyl, phenyl or phenyl-$C_1$-$C_4$-alkyl.

**12.** The compound of the formula

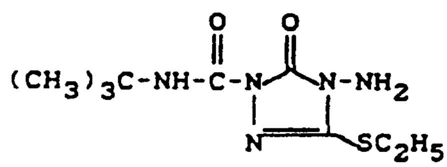

**13.** The compound of the formula

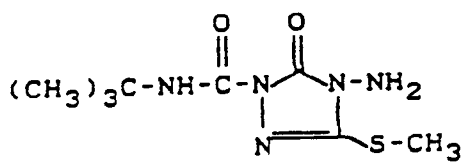

**14.** The compound of the formula

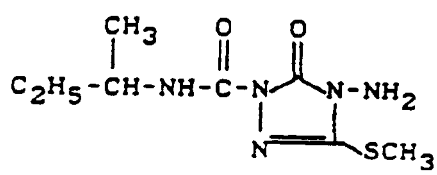

## Revendications

**1.** 4-amino-5-alkylthio-1,2,4-triazole-3-ones substituées de formule (I)

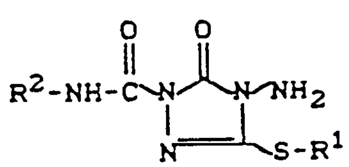

(I)

dans laquelle

$R^1$ représente un groupe alkyle, alcényle ou alcynyle, chacun à chaîne droite ou ramifiée et avec chacun jusqu'à 4 atomes de carbone et

$R^2$ est un groupe sec.-butyle, tertio-butyle, un groupe alkyle en $C_5$ à $C_{10}$, alcényle en $C_5$ à $C_{10}$ ou alcynyle en $C_5$ à $C_{10}$, chacun à chaîne droite ou ramifiée, un groupe alkyle, alcényle ou alcynyle avec chacun jusqu'à 10 atomes de carbone et portant chacun un substituant halogéno, cyano, cycloalkyle en $C_3$ à $C_6$, aryloxy de 6 à 10 atomes de carbone ou alkoxy en $C_1$ à $C_6$, un groupe alkyle en $C_1$ à $C_6$ portant un substituant pipéridyle ou morpholinyle, un groupe cyclopropyle, cyclobutyle, cyclopentyle ou cycloheptyle portant le cas échéant un substituant halogéno, aryle ayant 6 à 10 atomes de carbone, alkyle en $C_1$ à $C_6$ ou halogénalkyle en $C_1$ à $C_4$, un groupe cyclohexyle portant un substituant halogéno, alkyle en $C_1$ à $C_6$,

alcényle en $C_2$ à $C_4$ ou halogénalkyle en $C_1$ à $C_4$, un groupe phényl-(alkyle en $C_1$ ou $C_2$) ou un groupe naphtyl-(alkyle en $C_1$ ou $C_2$) portant le cas échéant dans le composant aromatique un substituant halogéno, alkyle en $C_1$ à $C_4$, halogénalkyle en $C_1$ ou $C_2$, alkoxy en $C_1$ à $C_4$, halogénalkoxy en $C_1$ ou $C_2$, alkylthio en $C_1$ à $C_4$, alkylsulfinyle en $C_1$ à $C_4$, alkylsulfonyle en $C_1$ à $C_4$, phénoxy et/ou phényle.

**2.** 4-amino-5-alkylthio-1,2,4-triazole-3-ones substituées de formule (I) suivant la revendication 1, dans laquelle

$R^1$ est un groupe méthyle, éthyle, propyle, isopropyle, butyle, isobutyle, sec.butyle, tertio-butyle, allyle, crotonyle ou propargyle et

$R^2$ est un groupe sec.-butyle, tertio-butyle, un groupe pentyle, hexyle, heptyle, octyle, pentényle, héxényle, heptényle, octényle, pentynyle, hexynyle, heptynyle ou octynyle, chacun à chaîne droite ou ramifiée, un groupe méthyle, éthyle, propyle isopropyle, butyle, isobutyle, sec.-butyle, tertio-butyle, pentyle, isopentyle, sec.-pentyle, tertio-pentyle, allyle, crotonyle ou propargyle portant chacun un substituant fluoro, chloro, bromo, cyano, cyclopropyle, cyclobutyle, cyclopentyle, cyclohexyle, phénoxy, méthoxy, éthoxy, propoxy, isopropoxy, butoxy, isobutoxy, sec.-butoxy ou tertio-butoxy, ou bien $R^2$ représente en outre un groupe méthyle, éthyle, n-propyle ou isopropyle portant chacun un substituant pipéridinyle ou morpholinyle, un groupe cyclopropyle, cyclobutyle ou cyclopentyle portant chacun le cas échéant un substituant fluoro, chloro, bromo, méthyle, trifluorométhyle, éthyle, propyle, isopropyle, butyle, isobutyle, sec.-butyle ou tertio-butyle, un groupe cyclohexyle portant un substituant fluoro, chloro, méthyle, trifluorométhyle, éthyle, propyle, isopropyle, butyle, isobutyle, sec.-butyle, tertio-butyle ou propargyle, $R^2$ représente en outre un groupe benzyle ou phényléthyle portant chacun facultativement dans le composant aromatique un substituant fluoro, chloro, bromo, méthyle, éthyle, propyle, isopropyle, trifluorométhyle, méthoxy, éthoxy, difluorométhoxy, trifluorométhoxy, méthylthio, méthylsulfinyle, méthylsulfonyle, phénoxy et/ou phényle.

**3.** Procédé de production de 4-amino-5-alkylthio- 1,2,4-triazole-3-ones substituées de formule (I)

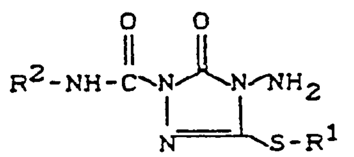

(I)

dans laquelle

$R^1$ représente un groupe alkyle, alcényle ou alcynyle, chacun à chaîne droite ou ramifiée et avec chacun jusqu'à 4 atomes de carbone et

$R^2$ est un groupe sec.-butyle, tertio-butyle, un groupe alkyle en $C_5$ à $C_{10}$, alcényle en $C_5$ à $C_{10}$ ou alcynyle en $C_5$ à $C_{10}$, chacun à chaîne droite ou ramifiée, un groupe alkyle, alcényle ou alcynyle avec chacun jusqu'à 10 atomes de carbone et portant chacun un substituant halogéno, cyano, cycloalkyle en $C_3$ à $C_6$, aryloxy de 6 à 10 atomes de carbone ou alkoxy en $C_1$ à $C_6$, un groupe alkyle en $C_1$ à $C_6$ portant un substituant pipéridyle ou morpholinyle, un groupe cyclopropyle, cyclobutyle, cyclopentyle ou cycloheptyle portant le cas échéant un substituant halogéno, aryle ayant 6 à 10 atomes de carbone, alkyle en $C_1$ à $C_6$ ou halogénalkyle en $C_1$ à $C_4$, un groupe cyclohexyle portant un substituant halogéno, alkyle en $C_1$ à $C_6$, alcényle en $C_2$ à $C_4$ ou halogénalkyle en $C_1$ à $C_4$, un groupe phényl-(alkyle en $C_1$ ou $C_2$) ou un groupe naphtyl-(alkyle en $C_1$ ou $C_2$) portant le cas échéant dans le composant aromatique un substituant halogéno, alkyle en $C_1$ à $C_4$, halogénalkyle en $C_1$ ou $C_2$, alkoxy en $C_1$ à $C_4$, halogénalkoxy en $C_1$ ou $C_2$, alkylthio en $C_1$ à $C_4$, alkylsulfinyle en $C_1$ à $C_4$, alkylsulfonyle en $C_1$ à $C_4$, phénoxy et/ou phényle,

caractérisé en ce que

(a) On fait réagir des hydrazones de formule générale (II)

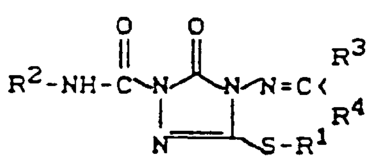

(II)

dans laquelle

$R^1$ et $R^2$ ont les définitions indiquées ci-dessus et

$R^3$ et $R^4$ représentent chacun, indépendamment l'un de l'autre, l'hydrogène, un groupe alkyle en $C_1$ à $C_6$, phényl-(alkyle en $C_1$ à $C_6$) ou phényle,
avec un acide, éventuellement en présence d'un diluant, ou bien
(b) On fait réagir des triazolones de formule générale (III)

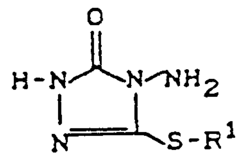

(III)

dans laquelle
$R^1$ a la définition indiquée ci-dessus, avec des isocyanates de formule générale (IV)

$R^2$ - N = C = O (IV)

dans laquelle
$R^2$ a la définition indiquée ci-dessus, éventuellement en présence d'un diluant et en la présence éventuelle d'une substance auxiliaire de réaction, ou bien
(c) On fait réagir des oxycarbonyltriazolones de formule générale (V)

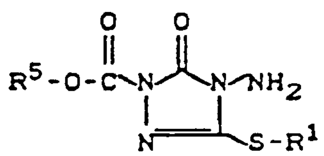

(V)

dans laquelle
$R^1$ a la définition indiquée ci-dessus et
$R^5$ est un groupe alkyle en $C_1$ à $C_6$, phényle ou phényl(alkyle en $C_1$ à $C_4$)
avec des amines de formule générale (VI)

$R^2$ - $NH_2$ (VI)

dans laquelle
$R^2$ a la définition indiquée ci-dessus, le cas échéant en présence d'un diluant et en la présence éventuelle d'une substance auxiliaire de réaction.

4. Compositions herbicides, caractérisées par une teneur en au moins une 4-amino-5-alkylthio-1,2,4-triazole-3-one substituée de formule (I) suivant la revendication 1.

5. Procédé pour combattre des plantes non désirées, caractérisé en ce qu'on fait agir des 4-amino-5-alkylthio-1,2,4-triazole-3-one substituées de formule (I) suivant la revendication 1 sur les plantes non désirées et/ou sur leur milieu.

6. Utilisation de 4-amino-5-alkylthio-1,2,4-triazole-3-ones substituées de formule (I) suivant la revendication 1 pour combattre des plantes non désirées.

7. Procédé de préparation de compositions herbicides, caractérisé en ce qu'on mélange des 4-amino-5-alkylthio-1,2,4-triazole-3-ones substituées de formule (I) suivant la revendication 1 avec des diluants et/ou des substances tensio-actives.

8. Hydrazones de formule (II)

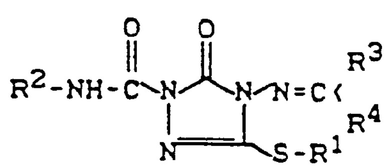

(II)

dans laquelle

$R^1$ représente un groupe alkyle, alcényle ou alcynyle, chacun à chaîne droite ou ramifiée et avec chacun jusqu'à 4 atomes de carbone et

$R^2$ est un groupe sec.-butyle, tertio-butyle, un groupe alkyle en $C_5$ à $C_{10}$, alcényle en $C_5$ à $C_{10}$ ou alcynyle en $C_5$ à $C_{10}$, chacun à chaîne droite ou ramifiée, un groupe alkyle, alcényle ou alcynyle avec chacun jusqu'à 10 atomes de carbone et portant chacun un substituant halogéno, cyano, cycloalkyle en $C_3$ à $C_6$, aryloxy de 6 à 10 atomes de carbone ou alkoxy en $C_1$ à $C_6$, un groupe alkyle en $C_1$ à $C_6$ portant un substituant pipéridyle ou morpholinyle, un groupe cyclopropyle, cyclobutyle, cyclopentyle ou cycloheptyle portant le cas échéant un substituant halogéno, aryle ayant 6 à 10 atomes de carbone, alkyle en $C_1$ à $C_6$ ou halogénalkyle en $C_1$ à $C_4$, un groupe cyclohexyle portant un substituant halogéno, alkyle en $C_1$ à $C_6$, alcényle en $C_2$ à $C_4$ ou halogénalkyle en $C_1$ à $C_4$, un groupe phényl-(alkyle en $C_1$ ou $C_2$) ou un groupe naphtyl-(alkyle en $C_1$ ou $C_2$) portant le cas échéant dans le composant aromatique un substituant halogéno, alkyle en $C_1$ à $C_4$, halogénalkyle en $C_1$ ou $C_2$, alkoxy en $C_1$ à $C_4$, halogénalkoxy en $C_1$ ou $C_2$, alkylthio en $C_1$ à $C_4$, alkylsulfinyle en $C_1$ à $C_4$, alkylsulfonyle en $C_1$ à $C_4$, phénoxy et/ou phényle,

$R^3$ et $R^4$ représentent chacun indépendamment l'un de l'autre de l'hydrogène, un groupe alkyle en $C_1$ à $C_6$, phényl-(alkyle en $C_1$ à $C_4$) ou phényle.

9. Oxycarbonyltriazolones de formule (V)

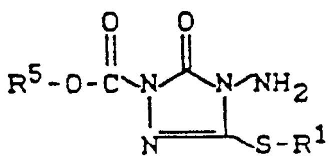

(V)

dans laquelle

$R^1$ désigne un groupe alkyle, alcényle ou alcynyle, chacun à chaîne droite ou ramifiée, ayant chacun jusqu'à 4 atomes de carbone et

$R^5$ est un groupe alkyle en $C_1$ à $C_6$, phényle ou phényl(alkyle en $C_1$ à $C_4$).

10. Triazolone-hydrazones de formule (VIII)

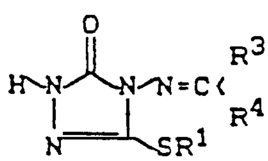

(VIII)

dans laquelle

$R^1$ désigne un groupe alkyle, alcényle ou alcynyle, chacun à chaîne droite ou ramifiée, avec chacun jusqu'à 4 atomes de carbone et,

$R^3$ et $R^4$ représentent chacun, indépendamment l'un de l'autre, l'hydrogène, un groupe alkyle en $C_1$ à $C_6$, phényl-(alkyle en $C_1$ à $C_4$) ou phényle.

11. Triazolones de formule (X)

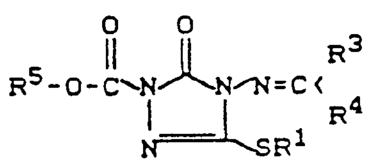

(X)

dans laquelle

$R^1$ désigne un groupe alkyle, alcényle ou alcynyle, chacun à chaîne droite ou ramifiée, avec chacun jusqu'à 4 atomes de carbone,

$R^3$ et $R^4$ représentent chacun, indépendamment l'un de l'autre, l'hydrogène, un groupe alkyle en $C_1$ à $C_6$, phényl-(alkyle en $C_1$ à $C_4$) ou phényle et

$R^5$ est un groupe alkyle en $C_1$ à $C_6$, phényle ou phényl-(alkyle en $C_1$ à $C_4$).

**12.** Le composé de formule

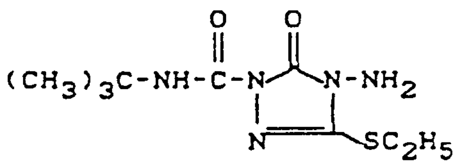

**13.** Le composé de formule

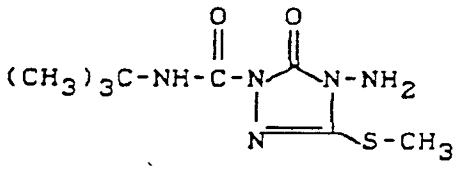

**14.** Le composé de formule